(19) Europäisches Patentamt
European Patent Office
Office européen des brevets

(11) EP 1 024 327 A1

(12) **EUROPEAN PATENT APPLICATION**
published in accordance with Art. 158(3) EPC

(43) Date of publication:
**02.08.2000 Bulletin 2000/31**

(21) Application number: **99939618.7**

(22) Date of filing: **20.08.1999**

(51) Int. Cl.$^7$: **F21V 33/00**, A61N 5/06

(86) International application number:
**PCT/JP99/04507**

(87) International publication number:
**WO 00/11397 (02.03.2000 Gazette 2000/09)**

(84) Designated Contracting States:
**DE FR GB NL**

(30) Priority: **21.08.1998 JP 23530598**
**21.08.1998 JP 23530698**
**21.08.1998 JP 23530798**
**07.05.1999 JP 12663199**

(71) Applicant:
**MATSUSHITA ELECTRIC INDUSTRIAL CO., LTD.**
**Kadoma-shi, Osaka 571-8501 (JP)**

(72) Inventors:
- **OHKUBO, Kazuaki**
  **Osaka 569-1142 (JP)**
- **HASHIMOTO, Kenjiro**
  **Osaka 535-0001 (JP)**
- **OTAKE, Shiro**
  **Osaka 572-0019 (JP)**

(74) Representative:
**Schwabe - Sandmair - Marx**
**Stuntzstrasse 16**
**81677 München (DE)**

## (54) RADIANT ENERGY IRRADIATION DEVICE

(57) A radiant energy irradiation device is provided with a means for irradiating an illuminating light containing radiation in a visible wavelength region and radiation in a specified wavelength region (for example, in the range of 600 to 1100 nm) that penetrates into a living body to maintain and promote biological functions. Radiation in this specified wavelength region can strengthen a biological immunity, activates an autonomic nerve system and maintain and promote biological functions. A radiation means for radiation in a visible wavelength region and a radiation means for radiation in a specified wavelength region may be integrated together or at least one of the two means may be independently provided.

FIG. 8

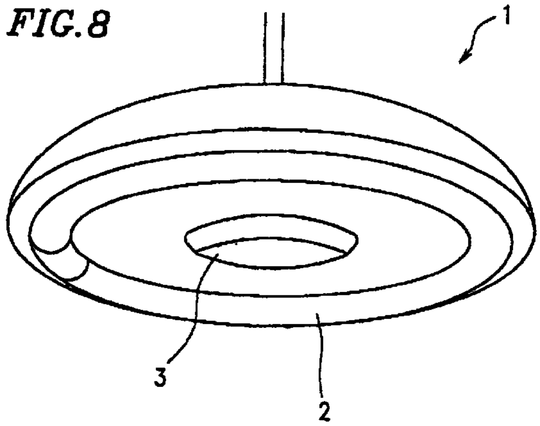




Printed by Xerox (UK) Business Services
2.16.7 (HRS)/3.6

## Description

### TECHNICAL FIELD

The present invention relates to a method and apparatus for radiating light (radiant energy) which realizes radiation of light (radiant energy) which is capable of unobtrusively maintaining/promoting biofunctions of a human in daily life.

The present invention further relates to an apparatus for radiating light (radiant energy) which also functions as an illumination apparatus having an ordinary illumination function in addition to the function of radiating light (radiant energy) as described above. The present invention also relates to an apparatus for radiating light (radiant energy) which also functions as a display apparatus having an image display function (display function) in addition to the function of radiating light (radiant energy) as described above (e.g., a display apparatus such as a TV image display apparatus, a computer display apparatus and a video game display apparatus).

### BACKGROUND ART

In recent years, the social environment around us has an increasing amount of stress factors, e.g., deterioration of the living environment such as the presence of incretion-disturbing substances, a fatigue feeling caused by the increasing commute distance and/or the increasing competition in the market, and information pollution. These not only affect humans but also other organisms such as pets and farm animals by damaging their biofunctions and thereby lowering the strength of their immune system and causing autonomic imbalance. Thus, there is a need for means to maintaining/promoting biofunctions of organisms such as humans in the environment in which they live.

Organisms have primarily maintained their biofunctions under sunlight. Accordingly, there have been beliefs that the biofunctions can be maintained/promoted by light irradiation, and it has long been said that daylight is needed for human health. Specifically, it is possible to externally promote an adjustment of the biofunctions if we can effectively stimulate biological sites related to the functions of the autonomic nervous system, the secretion function and the immune function by using near infrared radiation which can permeate into organisms.

However, for illumination in houses and offices, fluorescent lamps have been widely employed for their improved efficiencies. On the other hand, in recent years, workers have been living lives with little exposure to sunlight as their commute distance increases and their offices are moved underground or into tall buildings. Even ordinary people may have little exposure to sunlight during the *Tsuyu* season (during the rainy season) or during winter in high latitude regions.

On the other hand, watching TV, working with a display apparatus of a computer or an information apparatus, and playing a TV game using a display apparatus for a long period of time may cause mental stress in addition to eye fatigue. Such a stress has been known to not only cause mental fatigue but also lower the biofunctions such as the strength of the immune system (Reference 1: Report of Japanese Traffic/Preventive Medicine Research Foundation "Influence of Long Distance Driving and Short Distance Driving on $\alpha$-wave and NK Cell Activity", 1995).

In connection with the above, in recent researches, it has been reported that red light improves the NK (Natural Killer) cell activity which is an important part of the immune system (Reference 2: 19th conference of Japanese Society for Photomedicine and Photobiology, B7-43, "Study on Influence on NK Cell Activity of Irradiation of Forehead with Red-Color Light emitting Diode", 1997, Reference 3: Japanese Laid-Open Publication No. 9-84888 "Non-Invasive Method for Increasing Immunological Surveillance Ability and Device for Irradiating Forehead with Pulsed Light"). It is said that the cause of this phenomenon may be the stimulus by the deeply-permeating red light to a site in the organism related to the immune function such as the hypothalamus in the brain.

A NK cell is a cell which has an important roll in the immune system and is an important cell which attacks and kills cancer cells and viruses. However, mental and physical stress and aging reduce the amount and the activity of NK cells, thereby causing tumors and viral infections. Therefore, it is important to maintain/promote NK cell activity in daily life.

Particularly, it is expected that the strength of the immune system would be more improved under illumination by light such as sunlight which contains a large amount of red light and near infrared radiation, rather than under fluorescent lamp illumination which does not contain such radiation. Conversely, it is expected that NK cell activity is reduced over a long period of time if one lives under fluorescent lamp illumination which contains little red light and near infrared radiation without being exposed to sunlight. For example, an aged person who stays in his/her room and watches TV all day, deskwork in the office which involves many hours of computer use, TV games, and the like, is being harmed in terms of the strength of the immune system.

Figure 1 shows a spectral distribution (the distribution of the relative spectral radiation energies with respect to wavelengths) for each of the following commonly employed light sources: (a) a three-band type daylight fluorescent lamp; (b) a white light fluorescent lamp; (c) a 60 W silica light bulb; and (d) a light emitting diode (LED) having an emission peak wavelength of 660 nm that is equivalent to that used in Reference 2. Table 1 shows the illuminance (in lx) which is required for each of the above-described four light sources so that the irradiance at a wavelength equal to or greater than 635 nm on the forehead of a subject is equivalent to that shown in Reference 1, i.e., the illuminance (in lx) which is required to obtain an NK cell activity equivalent to that obtained by using an LED in Reference 1. Specifically, Table 1 shows, for each light source, the irradiance at a wavelength in the range of 635 nm to 1000 nm and the illuminance which is required to obtain, with a 30 minute irradiation, an NK cell activity equivalent to that obtained by a 30 minute irradiation at an illuminance of 80 lx by using an LED having an emission peak wavelength of 660 nm. The 635 nm wavelength is a half-width wavelength of the LED having an emission peak wavelength of 660 nm.

Table 1

| Illuminance required to obtain NK activity equivalent to that obtained by using LED | | |
|---|---|---|
| | Irradiance (635-1000 nm) $\mu W/cm^2/1000$ lx | Illuminance required for the same irradiance as that obtained by using LED 80 lx |
| LED(660 nm) | 167.3 | 80 lx (30 min) |
| Three-band type fluorescent lamp (daylight) | 5.2 | 2574 lx (30 min) |
| White light fluorescent lamp | 7.1 | 1885 lx (30 min) |
| Silica 60 W | 283.2 | 47 lx (30 min) |
| 635 nm is half-width wavelength of LED 660 nm | | |

Thus, a fluorescent lamp, either a daylight type fluorescent lamp or a white light type fluorescent lamp, has substantially no radiation at a wavelength equal to or greater than 700 nm, as shown in Figure 1, and cannot provide radiation in a red and near infrared region which provides the above-described desirable effects. The illuminance on a work plane (on a desk) obtained by an office illumination using a fluorescent lamp is typically about 1000 lx. However, as can be seen from Table 1, in order to obtain, by using a fluorescent lamp illumination, a radiation effect in a red and near infrared region (activation of NK cells) equivalent to that obtained by using an LED, the illuminance needs to be equal to or greater than about 2 times the value used in the above-described typical office illumination.

On the other hand, a silica light bulb (incandescent lamp) provides a sufficient radiation in a red and near infrared region, as shown in Figure 1, and thus can provide an effect of activating NK cells equivalent to that obtained by using an LED at an illuminance lower than that of an LED radiation. However, a silica light bulb has a poor efficiency (illuminance/input power) and is disadvantageous in terms of power conservation, and also has an adverse effect in terms of thermal radiation.

Reference 3 further reports that the effect of increasing the NK cell activity is more pronounced when light from an LED having a wavelength of 660 nm is radiated in a form of pulsed light of 0.5 Hz to 13 Hz for the purpose of inducing a wave. The apparatus arrangement disclosed in Reference 3 is intended to serve as a therapeutic device. As such, it is not a problem to irradiate a human with such red flashing light because the eyes of the human to be irradiated are closed. However, if much a principle (irradiation with red flashing light) is applied to a general purpose illumination apparatus, such a red region flashing frequency is very uncomfortable for a person who lives or works under the illumination, and may possibly cause epileptic seizure.

As described above, with the conventional illumination techniques, it is not possible to obtain a light source which can replace daylight, providing a sufficient radiation in a red and near infrared region which is effective for maintaining/promoting the strength of the immune system. While one may consider arranging a light source to surround a display section (screen) of a display apparatus for the purpose of obtaining a sufficient radiation in a red and near infrared region, such an apparatus arrangement (light source arrangement) is not preferred because it will make the observer uncomfortable by causing a glare and will also lower the productivity.

As described above with the conventional techniques, it is not possible to maintain/improve the NK cell activity of a person who watches TV under an artificial illumination over a long period of time or who does their work using a display, i.e., who cannot be sufficiently exposed to daylight. Therefore, with the conventional techniques, it is not possible to obtain a practical method and apparatus for radiating light (radiant energy) which is capable of maintaining/promoting biofunctions such as immune functions or functions of the autonomic nervous system, which are needed in our living environment with a lot of stress.

DISCLOSURE OF THE INVENTION

The present invention has been made to overcome the problems as described above, and has objectives of: (1) providing an apparatus and method for radiating light (radiant energy) which is capable of always realizing a healthy living condition irrespective of the living environment by unobtrusively irradiating in daily life an organism with light (radiant energy) (particularly, radiation in a red to near infrared wavelength range) which maintains/promotes biofunctions; (2) providing an apparatus for radiating light (radiant energy) which also functions as an illumination apparatus having a general illumination function in addition to the light (radiant energy) radiating function as described above: and (3) providing an apparatus for radiating light (radiant energy) which also functions as a display apparatus having an image display function (display function) in addition to the light (radiant energy) radiating function as described above, and thus is capable of maintaining/improving biofunctions of an organism who watches a display over a long period of time.

A radiant energy radiation apparatus of the present invention is a radiant energy radiation apparatus, comprising means for radiating illumination light for an illumination purpose, the illumination light containing radiation in a visible wavelength range and radiation in a predetermined wavelength range which permeates into an organism to maintain/promote biofunctions, thereby achieving the above-described object.

Preferably, the predetermined wavelength range is a range of 600 nm to 1100 nm.

For example, the predetermined wavelength range may maintain/promote the biofunctions by increasing a strength of an immune system of the organism.

Alternatively, radiation in the predetermined wavelength range may maintain/promote the biofunctions by activating an autonomic nervous system.

Radiation means for radiation in a visible wavelength range and radiation means for radiation in the predetermined wavelength range may be integrated together.

Alternatively, radiation means for radiation in a visible wavelength range and radiation means for radiation in the predetermined wavelength range may be independently provided from each other.

Preferably, on an irradiated plane to be irradiated with the illumination light, an irradiance at a wavelength in a range of 600 nm to 1100 nm is 0.1 $W/m^2$ or more.

Preferably, radiation in the predetermined wavelength range is radiation in a range of 600 nm to 1100 nm, and the radiation in the range of 600 nm to 1100 nm is radiated while being pulse-modulated at 0.5 to 13 Hz.

Preferably, on an irradiated plane to be irradiated with the illumination light, radiant energy of radiation at a wavelength in a range of 600 nm to 1100 nm is equal to or greater than 15% of radiant energy of radiation at a wavelength in a visible wavelength range of 380 nm to 780 nm.

Preferably, a radiant efficiency of radiation at a wavelength in a range of 600 nm to 1100 nm is equal to or greater than 0.001 W/lm.

Preferably, on an irradiated plane to be irradiated with the illumination light, radiant energy of radiation at a wavelength in a range of 1100 nm to 2.5 μm is smaller than radiant energy of radiation at a wavelength in a range of 600 nm to 1100 nm.

Preferably, the illumination light has a color of light which does not cause discomfort, and a deviation (duv) of the chromaticity of light from a Planckian locus in Commission Internationals de l'Eclairage (CIE) 1960 UCS chromaticity diagram is within ±0.01.

The above-described radiant energy radiation apparatus of the present invention may have a configuration of a discharge lamp, a fluorescent discharge lamp, an incandescent lamp, or a light source including a solid light emitting device.

Another radiant energy radiation apparatus of the present invention is a radiant energy radiation apparatus, comprising means for radiating radiation in a predetermined wavelength range which has a low visibility for a human and which deeply permeates into an organism to maintain/promote biofunctions, thereby achieving the above-described object.

Preferably, the predetermined wavelength range is a range of 600 nm to 1100 nm.

For example, radiation in the predetermined wavelength range may maintain/promote the biofunctions by increasing a strength of an immune system of the organism.

Alternatively, radiation in the predetermined wavelength range may maintain/promote the biofunctions by activating an autonomic nervous system.

Preferably, on an irradiated plane to be irradiated with radiation, an irradiance at a wavelength in a range of 700 nm to 1100 nm is 0.03 $W/m^2$ or more.

Preferably, radiation in the predetermined wavelength range is radiation in a range of 700 nm to 1100 nm, and radiation in the range of 700 nm to 1100 nm is radiated while being pulse-modulated at 0.5 to 13 Hz.

Preferably, on an irradiated plane to be irradiated with radiation, radiant energy of radiation at a wavelength in a range of 1100 nm to 2.5 μm is smaller than radiant energy of radiation at a wavelength in a range of 700 nm to 1100 nm.

The above-described radiant energy radiation apparatus of the present invention may have a configuration of a discharge lamp, a fluorescent discharge lamp, an incandescent lamp, or a light source including a solid light emitting device.

The above-described radiant energy radiation apparatus of the present invention may have an illumination function of providing illumination light for an illumination purpose.

Alternatively, the above-described radiant energy radiation apparatus of the present invention may have a display function of displaying a predetermined image. In such a case, for example, the predetermined image may be displayed by the means for radiating radiation in the predetermined wavelength range. Alternatively, display means for displaying the predetermined image may be further provided, and the means for radiating radiation in the predetermined wavelength range may be attached to the display means.

BRIEF DESCRIPTION OF THE DRAWINGS

Figure **1** shows a spectral distribution (the distribution of the relative spectral radiation energies with respect to wavelengths) for each of the following commonly employed light sources: (a) a three-band type daylight fluorescent lamp; (b) a white light fluorescent lamp; (c) a 60 W silica light bulb; and (d) a light emitting diode (LED) having an emission peak wavelength at 660 nm.

Figure **2** shows a spectral distribution (the distribution of the relative spectral radiation energies with respect to wavelengths) for each of test illumination light (illumination light IL+TR including near infrared radiation, and illumination light FL not including near infrared radiation) used in an experiment conducted by the present inventors.

Figure **3** shows a spectral distribution (the distribution of the relative spectral radiation energies with respect to wavelengths) for each of a three-band type fluorescent lamp (daylight) and a white light fluorescent lamp.

Figure **4** shows spectroscopic absorption characteristics of the main components of an organism, i.e., water and hemoglobin, in the vicinity of a near infrared wavelength range.

Figure **5** shows a spectral distribution (the distribution of the relative spectral radiation energies with respect to wavelengths) for an MFG fluorescent lamp which is configured according to the present invention.

Figure **6** shows a spectral distribution (the distribution of the relative spectral radiation energies with respect to wavelengths) for an ALF fluorescent lamp which is configured according to the present invention.

Figure **7** shows a distribution of relative spectral radiation energies for an MFG fluorescent lamp and an ALF fluorescent lamp, along with the results for an LED having an emission peak wavelength of 660 nm and a 60 W silica white light bulb.

Figure **8** schematically illustrates a configuration of a light radiation apparatus (lighting fixture) which is configured according to the present invention.

Figure **9** schematically illustrates a configuration of a display apparatus which is configured according to the present invention.

Figure **10** schematically illustrates another configuration of a display apparatus which is configured according to the present invention.

BEST MODE FOR CARRYING OUT THE INVENTION

In the course of achieving the present invention, the present inventors examined by way of experiment, what differences would occur in the functions of the autonomic nervous system and/or secretory functions of an organism between under illumination light containing near infrared radiation and under illumination light not containing near infrared radiation. Before the detailed description of embodiments of the present invention, the results of the experiments conducted by the present inventors will be discussed below.

Illumination light containing near infrared radiation (hereinafter, referred to as "illumination light IL+TR") was produced by an incandescent lamp. The incandescent lamp illumination light (IL) was passed through a heat ray absorbing filter (TR) so as to prevent a thermal effect of far infrared radiation contained in the incandescent lamp from influencing an organism. On the other hand, illumination light not containing near infrared radiation (hereinafter, referred to as "illumination light FL") was produced by a fluorescent lamp which can provide illumination light having the same color of light as that of the illumination light containing near infrared radiation ("illumination light IL+TR"). A spectral distribution (the distribution of the relative spectral radiation energies with respect to wavelengths) for each of the test illumination light is shown in Figure **2**.

Each of the illumination light FL and the illumination light IL+TR was set so that the illuminance would be about 200 lx on the head of the irradiated subject. This value is within an illuminance range of 150 to 300 lx which is recommended in the Japanese Industrial Standard (JIS) Z9110 "Recommended level of illumination" for conversation/entertainment in a living room.

The illumination light IL+TR and the illumination light FL were switched to each other by changing the light source of a lighting fixture arranged in the experiment room. Thus, not only the illuminance and the color of light, but also the surrounding light environment, were set to be the same between the test illumination light so that it is possible to extract the influence of the presence/absence of near infrared radiation on biofunctions.

Subjects comprised eight males in their 20's - 50's. Each subject wearing clothing with his head exposed, entered the experiment room which was illuminated by a fluorescent lamp (whose light was not illumination light to be tested but had the same color of light as the illumination light FL and did not contain near infrared radiation), and sat quietly in the room. Then, the blood pressure (highest blood pressure), the heart rate, the noradrenaline concentration in blood, cortisol concentration in blood, and the NK cell activity of the subject were measured. After the measurement, the fluorescent lamp illumination light was turned off, and then the subject was exposed to either one of the test illumination light (the illumination light IL+TR or the illumination light FL). The subject was instructed to sit quietly under the test illumination light for 30 minutes. After 30 minutes, the heart rate, the blood pressure (highest blood pressure), the noradrenaline concentration in blood, cortisol concentration in blood, and the NK cell activity of the subject were measured again.

The above procedure was repeated for different test illumination light, and was repeated for the same subject for repeated measurements. Then, a statistical analysis was performed to determine for each test illumination light the difference between each measurement value before the irradiation with the test illumination light and that after the irradiation with the test illumination light. The results are shown in Table 2.

Table 2

| | Illumination light FL | Illumination light IL+TR | |
|---|---|---|---|
| Heart rate | 1.4±2.0 | -3.6±1.5 | [beats/min] |
| Highest blood pressure | -1.6±3.5 | -3.8±3.1 | [mmHg] |
| Lowest blood pressure | 0.1±3.2 | -3.5±1.7 | [mmHg] |
| Noradrenaline concentration in blood | -30±17 | -44±14 | [pg/ml] |
| Cortisol concentration in blood | -1.6±0.7 | -1.6±0.7 | [μg/dl] |
| NK cell activity | -4.2±1.9 | -2.5±1.7 | [%] |

Table 2 shows that under the illumination light FL, the heart rate and blood pressure (highest blood pressure) before the exposure are not significantly different from those after the exposure. Under the illumination light IL+TR, however, the heart rate and the blood pressure (maximum blood pressure) were significantly reduced by the exposure. While the noradrenaline concentration in blood was reduced by the exposure to either the illumination light IL+TR or the illumination light FL, it was slightly more reduced by the exposure to the illumination light IL+TR than by the exposure to the illumination light FL. The cortisol concentration in blood was reduced by the exposure to either the illumination light IL+TR or the illumination light FL, and there was no significant difference in the level of reduction between the respective illumination light. While the NK cell activity was reduced by the exposure to either the illumination light IL+TR or the illumination light FL, the NK cell activity value after the illumination light IL+TR was slightly higher than that after the illumination light FL.

As described above, the blood pressure and the heart rate were reduced by the exposure to the illumination light IL+TR, suggesting that near infrared radiation might have activated the parasympathetic nerve. Noradrenaline in blood has a function of increasing the heart rate. The greater reduction in the noradrenaline concentration in blood obtained by the exposure to the illumination light IL+TR corresponds to the suppression of the heart rate by the irradiation with the illumination light IL+TR.

More cortisol is secreted in blood as the stress increases. That there was no difference in the cortisol concentration in blood after exposure between the respective test illumination light suggests that there was no influence of a stress due to a task, or the like, for the respective test illumination light and that the experiment was appropriate. Moreover, since the NK cell activity value was higher for the illumination light IL+TR, it can be said that near infrared radiation has an effect of improving the strength of the immune system.

Thus, near infrared radiation is useful in maintaining/promoting biofunctions, e.g., improving the strength of the immune system, activating the functions of the autonomic nervous system, and adjusting the secretion function, and has an effect of increasing the NK cell activity of an organism.

Figure **3** shows an example of a spectral distribution for each of a three-band type fluorescent lamp (daylight) and a white light fluorescent lamp, which exemplify general purpose illumination light sources widely used in offices and houses. Either of these fluorescent lamps has substantially no radiation at a wavelength equal to or greater then 635 nm.

The illuminance required to obtain an irradiance at a wavelength equal to or greater than 635 nm which is equivalent to that obtained by using the illumination light IL+TR is 3400 lx for illumination light of a three-band type fluorescent lamp and 2700 lx for illumination light of a white light fluorescent lamp. Thus, it is required to provide an illuminance that is several times the value (750 to 1500 lx) recommended for the illuminance on a work plane under office illumination according to JIS Z 9110. However, this illuminance is too high for house illumination and unacceptable as it causes adverse influences such discomfort by a glare, or the like.

On the other hand, an incandescent lamp (silica light bulb) provides a sufficient radiation at a wavelength equal to or greater than 635 nm which is a component of the illumination light IL+TR and corresponds to a red to near infrared region, as already discussed above with reference to Figure **1**. However, as described above, a incandescent lamp has a poor efficiency (illuminance/input power) and it generates heat to increase the air conditioning load, thereby hindering energy conservation. Moreover, with an incandescent lamp, far infrared radiation in a range of 1100 nm to 2.5 μm generates heat, which may even increase the stress on the organism, in which case an incandescent lamp may have a negative effect on the object of maintaining/promoting biofunctions.

As described above, according to the experiments conducted by the present inventors, red and near infrared radiation in a wavelength range of 635 nm to 1100 nm, with which the head was irradiated, permeates into the organism to stimulate sites in the head which are related to the immune function and the functions of the autonomic nervous system. This maintains/promotes biofunctions and improves the NK cell activity of the organism.

The inside of an organism is surrounded by water and hemoglobin in blood which respectively have absorption characteristics as shown in Figure **4** with respect to various wavelengths. Therefore, particularly, radiation in a wavelength range of 700 nm to 1100 nm where the absorption of both water and hemoglobin is small (i.e., a wavelength range which serves as a "organism window") more efficiently permeates into the organism to stimulate the hypothalamus in the brain. Moreover, radiation in an infrared wavelength range corresponding to the organism window is hardly perceived by an eye. When light in a visible wavelength range is modulated at a frequency around 10 Hz, as shown in Reference 3, uncomfortable flickering will be perceived which may cause epileptic seizure. On the contrary, when light emitted from a light source in this infrared wavelength range is modulated at a frequency around 10 Hz, as shown in Reference 3, such uncomfortable flickering will not occur.

Thus, according to the present invention, a conventional discharge lamp, a conventional fluorescent lamp, or a conventional illumination apparatus (light radiation apparatus) using the same as its light source, is modified by providing the light source with radiation having a wavelength component in a red to near infrared region which provides the effects as described above, in addition to an emission spectrum (radiation in a visible wavelength range) inherent to the light source itself. Therefore, a person who lives or works under the illumination can be exposed to a sufficient amount of radiation which realizes a sufficient NK cell activity.

Instead of providing the light source itself with radiation having a wavelength component in a red to near infrared region, a separate radiation source having a wavelength spectrum in the red to near infrared region may alternatively be added to the light source (radiation source) for the inherent emission spectrum (radiation in a visible wavelength range). In such a case, similar effects can be obtained also when the light source (radiation source) for radiation in a visible wavelength range and the light source (radiation source) for radiation in a red to near infrared region are arranged, for example, on a wall surface in a room and the opposing wall surface in the room, respectively, or on the ceiling of the room near one wall surface and on the ceiling of the room near the opposing wall surface, respectively.

With any one of the above arrangements, it is possible to provide an organism in daily life with radiation in a wavelength range capable of maintaining/improving biofunctions, e.g., improving the strength of the immune system and activating the functions of the autonomic nervous system, or to maintain/improve the NK cell activity, if the apparatus and method for radiating light (radiant energy) of the present invention is used. The apparatus for radiating light (radiant energy) of the present invention can be configured as a general purpose illumination apparatus. By providing an illumination apparatus having such a configuration, it is possible to, for example. maintain/improve biofunctions or the NK cell activity of a person who inevitably lives or works under an artificial illumination over a long period of time and thus cannot be sufficiently exposed to daylight, or to provide a person always with a sufficient amount of radiation irrespective of the weather, the regionality, the season of the year, etc. Thus, according to the present invention, it is possible to unobtrusively maintain/improve biofunctions or the NK cell activity in daily life with irradiation with light (radiant energy) irrespective of the living environment.

Alternatively, it is of course possible to configure a light (radiant energy) radiation apparatus so as to function as a therapeutic device of the present invention.

As will be described later in detail, the light source of the present invention may be configured to radiate an alternating or pulsed wave with a frequency of 0.5 Hz to 13 Hz.

Alternatively, the light (radiant energy) radiation apparatus of the present invention can be further provided with an image display function (display function) so that it functions as a display apparatus. For example, the light source (light radiation apparatus) of the present invention can be arranged around a screen (display section) of a conventional display apparatus such as a TV or a computer display apparatus, so that it is possible to efficiently irradiate the face of the viewer or the worker with light in a red to near infrared region when they have their face in the vicinity of the screen as they watch TV or do work with the display. Thus, it is possible to maintain/improve the NK cell activity in the body of a person who watches a display over a long period of time. e.g., a person who watches TV or who does OA (Office Automation) work. The location around the screen of a display apparatus where the light source (light radiation apparatus) of the present invention is to be arranged may be in the display section (screen) of the display apparatus or around any other component, e.g., along the frame of the display apparatus or around the screen (display section) of the display apparatus. Alternatively, it is possible to control such radiation by, for example, applying a fluorescent substance having an emission spectrum at a wavelength equal to or greater than 700 nm as a part of the fluorescent substance on the CRT screen of the display so as to emit a signal which radiates light capable of maintaining/promoting biofunctions in combination with a video signal, thereby adding a spectrum in the 700 nm to 1100 nm wavelength range to the emission from the display screen.

The upper limit of the radiation wavelength range is desirably set to be less than or equal to 1100 nm, which is the upper limit of the "organism window" shown in Figure **4**. When the illumination light contains far infrared radiation whose wavelength exceeds 1100 nm, the thermal effect of far infrared radiation (a thermal effect occurring due to the optical absorption by water in the body) may cause a stress on the organism, thereby lowering the effect of maintaining/promoting biofunctions. In this regard, the present inventors have experimentally confirmed that a light source not containing radiation of 1100 nm or more has a greater effect of reducing the heart rate than a light source containing radiation in a frequency range of 1100 nm to 2.5 μm. This shows that it is possible to ensure the effect of maintaining/promoting biofunctions by reducing radiant energy in a wavelength range of 1100 nm to 2.5 μm to be, at least, smaller than the effective radiant energy in a wavelength range of 600 nm to 1100 nm.

The lower limit of the radiation wavelength range which exhibits a desirable effect of maintaining/promoting biofunctions is, in principle, 600 nm. As shown in Figure **4**, for a wavelength smaller than 600 nm, the absorption by hemoglobin will be substantial, whereby effective radiation cannot be expected. Moreover, in order to configure a practical light source according to the present invention, the radiation wavelength is preferably set to be equal to or greater than 635 nm. An existing three-band type fluorescent lamp contains a large amount of radiation in a wavelength range of 600 nm to 635 nm. Therefore, in order to configure a light source which is distinguished from existing light sources and more effective according to the present invention, it is preferred to set the lower limit of the radiation wavelength to be 635 nm. Moreover, it is said that light at a wavelength of 660 nm has an effect of promoting the strength of the immune system. Therefore, such an effect can be expected by including radiation at this frequency. However, as shown in Figure **4**, for a wavelength smaller than 700 nm, the absorption coefficient by hemoglobin is still greater than that for a wavelength in the organism window. Therefore, a radiated energy can be more efficiently absorbed into the organism by using a wavelength range of 700 nm or more.

Figure **4** further shows that particularly for a wavelength range of 800 nm to 1000 nm, the optical absorption coefficient by water and the optical absorption coefficient by hemoglobin are both sufficiently small. This shows that a radiated energy can be more efficiently absorbed into the organism by setting the radiation wavelength in this wavelength range of 800 nm to 1000 nm.

In the case of a color display apparatus, emission in the vicinity of 635 nm is sometimes used to provide a strong red color. When red color light in the vicinity of 635 nm is radiated, the effect of maintaining/improving biofunctions can be expected, but the effect may be canceled out by a stress caused by watching such a stimulative red color. Therefore, in order to obtain an effect of non-visible light as in the case of applying the present invention to a display apparatus (when providing the apparatus with a display function), it is possible to use light in a wavelength range of 700 nm to 1100 nm whose visibility is lower than that of a visible range wavelength or which will not be perceived as light, thereby maintaining/promoting biofunctions without the user observing any unusual color.

One way to configure the light (radiant energy) radiation apparatus of the present invention which includes radiant energy in a wavelength range of 600 nm to 1100 nm which has an effect of maintaining/promoting biofunctions, and wherein radiant energy in a wavelength range of 1100 nm to 2.5 μm is smaller than radiant energy in a wavelength range of 600 nm to 1100 nm, is to provide radiation in a wavelength range of 600 nm to 1100 nm in the emission spectrum of the light source itself.

Where the light source is a discharge lamp, it is possible to select a substance to be enclosed in the lamp such that radiation in the wavelength range of 600 nm to 1100 nm can be obtained in plasma emission. Even when radiant energy in the wavelength range of 1100 nm to 2.5 μm is larger than radiant energy in the wavelength range of 600 nm to 1100 nm in the spectral radiant energy distribution of the plasma emission, a light radiation apparatus using the discharge lamp as its light source can be provided with a heat ray absorbing filter attached to the radiation window of the apparatus so that radiant energy in the wavelength range of 1100 nm to 2.5 μm is smaller than radiant energy in the wavelength range of 600 nm to 1100 nm on the irradiated plane. A discharge lamp having a better radiant efficiency can be obtained when the discharge lamp itself has radiation in the wavelength range of 600 nm to 1100 nm and has a spectral radiant energy distribution such that radiant energy in the wavelength range of 1100 nm to 2.5 μm is smaller than radiant energy in the wavelength range of 600 nm to 1100 nm.

Where the light source is a fluorescent discharge lamp, it is possible to select a fluorescent substance with which radiation in the wavelength range of 600 nm to 1100 nm can be obtained. Moreover, even when the fluorescent substance has a spectral radiant energy distribution such that radiant energy in the wavelength range of 1100 nm to 2.5 μm is larger than radiant energy in the wavelength range of 600 nm to 1100 nm, a light radiation apparatus using the fluorescent discharge lamp as its light source can be provided with a heat ray absorbing filter attached to the radiation window of the apparatus so that radiant energy in the wavelength range of 1100 nm to 2.5 μm is smaller than radiant energy in the wavelength range of 600 nm to 1100 nm on the irradiated plane. A fluorescent discharge lamp having a better radiant efficiency can be obtained when the fluorescent discharge lamp itself has radiation in the wavelength range of 600 nm to 1100 nm and has a spectral radiant energy distribution such that radiant energy in the wavelength range of 1100 nm to 2.5 μm is smaller than radiant energy in the wavelength range of 600 nm to 1100 nm.

Where the light source is an incandescent lamp, radiant energy in the wavelength range of 1100 nm to 2.5 μm is typically larger than radiant energy in the wavelength range of 600 nm to 1100 nm, as can be seen from the spectral distribution characteristics described above with reference to Figure **1**. Therefore, a light radiation apparatus using an incandescent lamp as its light source requires a configuration on the irradiated plane such that radiant energy in the wavelength range of 1100 nm to 2.5 μm is smaller than radiant energy in the wavelength range of 600 nm to 1100 nm. For example, a filter having spectral transmission characteristics such that the amount of radiation in the wavelength range of 600 nm to 1100 nm transmitted is larger than the amount of radiation in the wavelength range of 1100 nm to 2.5 μm transmitted can be attached to the radiation window of the light radiation apparatus (i.e., a window through which light emitted from the incandescent lamp as a light source is externally output). Alternatively, the spectral radiation spectrum of the incandescent lamp itself can be provided with characteristics such that radiant energy in the wavelength range of 1100 nm to 2.5 μm is smaller than radiant energy in the wavelength range of 600 nm to 1100 nm by, for example, employing as a glass forming the bulb of the incandescent lamp a material having spectral transmission characteristics such that the amount of radiation in the wavelength range of 600 nm to 1100 nm transmitted is larger than the amount of radiation in the wavelength range of 1100 nm to 2.5 μm transmitted, or by providing a multilayer interference film which selectively transmits radiant energy in the wavelength range of 600 nm to 1100 nm on the glass surface.

Alternatively, similar effects can be obtained also when the light (radiant energy) radiation apparatus is configured with a light source which is a solid light emitting device such as a light emitting diode, a semiconductor laser, an electroluminescence device, and the like, which primarily has radiant energy in the wavelength of 600 nm to 1100 nm. Even when such a device includes a large amount of radiation in the wavelength range of 1100 nm to 2.5 μm, a light radiation apparatus using such a device as its light source can be provided with a heat ray absorbing filter attached to the radiation window of the apparatus, as described above, so that radiant energy in the wavelength range of 1100 nm to 2.5 μm is smaller than radiant energy in the wavelength range of 600 nm to 1100 nm on the irradiated plane.

In the above-described experiments conducted by the present inventors, the irradiance on the irradiated plane in the wavelength range of 635 nm to 1100 nm was 0.63 $W/m^2$ for the illumination light IL+TR which exhibited a significant effect of maintaining/promoting biofunctions, and 0.05 $W/m^2$ for the illumination light FL which did not exhibit a significant effect. Since the factor of the measurement error due to irradiance variations is at most 2 or less, a light (radiant energy) radiation method capable of providing a significant effect of maintaining/promoting biofunctions can be obtained by setting the irradiance on an organism in the wavelength range 600 nm to 1100 nm to 0.1 $W/m^2$ or more, based on the above-described results. It is preferred that radiant energy in the wavelength range of 1100 nm to 2.5 μm is smaller than radiant energy in the wavelength range of 600 nm to 1100 nm.

In the experiments conducted by the present inventors, in the case where the present inventors attempted to obtain effects by non-visible light radiation, the irradiance on the irradiated plane in the wavelength range of 700 nm to 1100 nm was 0.37 $W/m^2$ for the illumination light IL+TR which exhibited a significant effect of maintaining/promoting biofunctions, and 0.017 $W/m^2$ for the illumination light FL which did not exhibit a significant effect. In view of these values, it can be seen that a light (radiant energy) radiation method capable of providing a significant effect of maintaining/promoting biofunctions can be obtained by setting the irradiance on an organism in the wavelength range 700 nm to 1100 nm to 0.03 $W/m^2$ or more. Again, it is preferred that radiant energy in the wavelength range of 1100 nm to 2.5 μm is smaller than radiant energy in the wavelength range of 700 nm to 1100 nm.

The upper limit of the irradiance is set to a limit value of $1\times10^5$ $W/m^2$ with which biological tissues such as the lens and the cornea would not be damaged.

As described above, in order to obtain a light (radiant energy) radiation apparatus capable of providing a significant affect of maintaining/promoting biofunctions. the intensity or the arrangement of the red to near infrared region radiation source is set such that the irradiance on the irradiated plane in the wavelength range of 600 nm to 1100 nm is 0.1 $W/m^2$ or more (or the irradiance on the irradiated plane in the wavelength range of 700 nm to 1100 nm is 0.03 $W/m^2$ or more). With a radiant energy radiation apparatus having such a configuration, it is possible to maintain/promote biofunctions of an organism irradiated by the apparatus.

As described above, a visible range wavelength can be added to radiation whose spectral energy distribution on the irradiated plane is such that the irradiance in the wavelength range of 600 nm to 1100 nm is 0.1 $W/m^2$ or more (or the irradiance on the irradiated plane in the wavelength range of 700 nm to 1100 nm is 0.03 $W/m^2$ or more) and such that radiant energy in the wavelength range of 1100 nm to 2.5 μm is smaller than radiant energy in the wavelength range of 600 nm to 1100 nm. In such a case, it is possible to obtain a light (radiant energy) radiation method capable of providing illumination light while maintaining/promoting biofunctions. A light (radiant energy) radiation apparatus for such light (radiant energy) radiation can be obtained as follows. The light (radiant energy) radiation apparatus is provided with a spectral energy distribution which includes visible light and radiation in a red to near infrared region in the wavelength range of 600 nm to 1100 nm, and configured so as to output radiation light such that radiant energy in the wavelength range of 1100 nm to 2.5 μm is smaller than radiant energy in the wavelength range of 600 nm to 1100 nm. Moreover, the light source is arranged so that at least the irradiance in the wavelength range of 600 nm to 1100 nm on the irradiated plane (e.g., the head, which is the center for maintaining/promoting biofunctions) is 0.1 $W/m^2$ or more (or the irradiance on the irradiated plane in the wavelength range of 700 nm to 1100 nm is 0.03 $W/m^2$ or more). If necessary, it is possible to increase the number of radiation sources and/or the input power to increase the radiation intensity so as to obtain an irradiance in the above-described range at least on the irradiated plane. A light (radiant energy) radiation apparatus having such a configuration can be used to maintain/promote the biofunctions while providing illumination on the object to be viewed.

The recommended illuminance for indoor illumination is defined in JIS Z 9110, for example, to be 150 lx or more for conversation/entertainment in a living room. Assuming that the light loss factor is 70%, radiant energy in the wavelength range of 600 nm to 1100 nm can be 0.1 $W/m^2$ or more with an illuminance equal to or greater than 100 lx if the radiant energy per unit photometric amount (i.e., the radiant efficiency) in the wavelength range of 600 nm to 1100 nm is equal to or greater than 0.001 W/lm or more. In order to realize a light (radiant energy) radiation apparatus having the characteristics as described above, where the light source is a discharge lamp, a substance to be enclosed in the lamp can be selected so that the plasma emission will radiate radiant energy in the wavelength range of 600 nm to 1100 nm in addition to visible light and that the radiant energy per unit photometric amount in the wavelength range of 600 nm to 1100 nm is 0.001 W/lm or more. Where the light source is a fluorescent discharge lamp, it is possible to select a fluorescent substance such that the light source will radiate radiant energy in the wavelength range of 600 nm to 1100 nm in addition to visible light and that the radiant energy per unit photometric amount in the wavelength range of 635 nm to 1100 nm is 0.001 W/lm or more. As described above, a heat ray absorbing filter, or the like, can be used if necessary in order to obtain a desirable spectral radiant energy distribution for either a discharge lamp or a fluorescent discharge lamp.

For indoor illumination, any uncomfortable color of light may cause a stress and adversely affect the biofunctions. This can be avoided by designing the light (radiant energy) radiation method such that: the irradiance on the irradiated plane in the wavelength range of 600 nm to 1100 nm is 0.1 $W/m^2$ or more; the radiant energy per unit photometric amount in the wavelength range of 600 nm to 1100 nm is 0.001 W/lm or more: radiant energy in the wavelength range of 1100 nm to 2.5 μm is smaller than radiant energy in the wavelength range of 635 nm to 1100 nm; and a color of light which is not uncomfortable is used. Uncomfortable colors of light include, for example, primary colors such as very strong red or blue. Such color of light should be avoided. A light (radiant energy) radiation apparatus in which the color of light is not uncomfortable can be obtained by: providing the light (radiant energy) radiation apparatus with optical means for correcting the color of light of the light source used; or by suitably selecting a substance to be enclosed in a lamp (in the case of a discharge lamp) or a fluorescent substance (in the case of a fluorescent discharge lamp) so that the light source itself has a color of light which is not uncomfortable. Again, as described above, a heat ray absorbing filter, or the like, can be used if necessary in order to obtain a desirable spectral radiant energy distribution.

For example, as a color of light for indoor illumination, white has been accepted. A person who is in the room would not feel uncomfortable at least by selecting such a color of light. Therefore, a light (radiant energy) radiation method for use in such cases can be obtained by setting the characteristics of radiated light so that: the irradiance on the irradiated plane in the wavelength range of 600 nm to 1100 nm is 0.1 $W/m^2$ or more; the radiant energy per unit photometric amount in the wavelength range of 600 nm to 1100 nm is 0.001 W/lm or more: radiant energy in the wavelength range of 1100 nm to 2.5 µm is smaller than radiant energy in the wavelength range of 600 nm to 1100 nm; and the deviation (duv) of the chromaticity of light from a Planckian locus in Commission Internationale de l'Eclairage (CIE) 1960 UCS chromaticity diagram is within ±0.01. A light (radiant energy) radiation apparatus in which the chromaticity of the radiated light is within such a range can be obtained by: providing the light (radiant energy) radiation apparatus with optical means for correcting the color of light of the light source used; or by suitably selecting a substance to be enclosed in a lamp (in the case of a discharge lamp) or a fluorescent substance (in the case of a fluorescent discharge lamp) so that the color of light of the light source itself has such a chromaticity. Again, as described above, a heat ray absorbing filter, or the like, can be used if necessary in order to obtain a desirable spectral radiant energy distribution.

When the color of light from the light source has a deviation (duv) of the chromaticity of light from a Planckian locus in CIE 1960 UCS chromaticity diagram which is outside the range of +0.01 to -0.01, the difference between the color of light and that of ordinary lighting will be substantial, thereby resulting in an increased discomfort for certain living environments or certain people and thus a stress associated therewith may lower the NK cell activity of the organism. Therefore, the color of light from the light source is preferable set so that the deviation (duv) of the chromaticity of light from a Planckian locus in CIE 1960 UCS chromaticity diagram is within the range of +0.01 to -0.01 (i.e., within ±0.01), as described above.

In the above description, the value ranges for irradiance on the irradiated plane and the radiant energy per unit photometric amount (i.e., the radiant efficiency) in the wavelength range of 600 nm to 1100 nm have been discussed. For the wavelength range of 700 nm to 1100 nm, the irradiance on the irradiated plane can be set to 0.03 $W/m^2$ or more, as described above, whereas the radiant energy per unit photometric amount (i.e., the radiant efficiency) on the irradiated plans can be set to be 0.0003 W/lm or more.

As described above, the method and apparatus for radiating light (radiant energy) of the present invention can provide radiation or illumination light capable of maintaining/promoting biofunctions, e.g., improving the strength of the immune system or activating the functions of the autonomic nervous system. Moreover, the site in the brain which controls the parasympathetic nerve of the autonomic nervous system is different from the site in the brain which controls the sympathetic nerve. Therefore, it is possible to selectively render dominant one of the parasympathetic nerve and the sympathetic nerve by changing the spectral composition, the intensity, the radiation direction, and the like, of the radiated light within the radiation conditions preferred in the present invention.

As described above, the apparatus and method for radiating light (radiant energy) of the present invention can be used to maintain/promote the biofunctions of a human using radiation of light (particularly, radiation in the red to near infrared region) so as to realize a healthier condition. Moreover, the method and apparatus of the present invention can also be used with other animals and/or plants such as pets and farm animals for the purpose of improving the strength of the immune system and/or activating the functions of the autonomic nervous system.

Several embodiment of the present invention will now be described with reference to the accompanying drawings.

(First Embodiment)

Figure 5 shows the emission spectrum of a fluorescent lamp which is provided as an exemplary light source to be mounted on a light radiation apparatus (lighting fixture) according to the present invention. The fluorescent lamp is an MFG fluorescent lamp which is provided by mixing a manganese activating magnesium fluorogermanate fluorescent substance ($3.5MgO \cdot 0.5MgF_2 \cdot GeO_2$:Mn, hereinafter referred to as "MFG") with a rare earth fluorescent substance used in conventional three-band type fluorescent lamps, and applying the obtained mixture on the lamp. The MFG fluorescent lamp of the present invention appears to be the same as a conventional fluorescent lamp, but the obtained emission spectrum is different from those of conventional fluorescent lamps.

The amount of radiation per unit photometric amount (radiant efficiency) in the wavelength range of 600 nm to 1100 nm of the above-described MFG fluorescent lamp which is obtained according to the present invention in 0.0025 W/lm. Using this MFG fluorescent lamp, the irradiance on the irradiated plane in the wavelength range of 600 nm to 1100 nm can be 0.1 $W/m^2$ or more by setting the illuminance to be 40 lx or more. When the illuminance is set to be 250 lx or more, effects similar to those obtained by using the above-described illumination light IL+TR can be expected. Moreover, the MFG fluorescent lamp does not have radiation at a wavelength equal to or greater than 1100 nm (far infrared radiation). Therefore, it is possible to prevent the thermal stress caused by far infrared radiation from adversely influencing the effect of maintaining/promoting the biofunctions provided by red to near infrared radiation.

However, the above-described MFG fluorescent lamp also does not include radiation at a wavelength of 700 nm or more. Therefore, the radiant efficiency on the irradiated plane in the wavelength range of 700 nm to 1100 nm is 0.00005 W/lm.

The chromaticity of the color of light provided by the above-described MFG fluorescent lamp is (x,y) = (0.4485, 0.4172) ,which is within ±0.01 from the blackbody radiation locus in the CIE 1960 UCS coordinate system. Therefore, the color of light is white, and thus the illumination light will cause no discomfort and will not inhibit the effect of maintaining/promoting biofunctions provided by red to near infrared radiation.

Figure **6** shows the emission spectrum of a fluorescent lamp which is provided as another exemplary light source to be mounted on a light radiation apparatus (lighting fixture) according to the present invention. The fluorescent lamp is an ALF fluorescent lamp which is provided by mixing an iron activating lithium aluminate fluorescent substance ($LiAlO_2$:Fe, hereinafter referred to as "ALF") with a rare earth fluorescent substance used in conventional three-band type fluorescent lamps, and applying the obtained mixture on the lamp. The ALF fluorescent lamp of the present invention also appears to be the same as a conventional fluorescent lamp, but the obtained emission spectrum is different from those of conventional fluorescent lamps.

The amount of radiation per unit photometric amount (radiant efficiency) in the wavelength range of 600 nm to 1100 nm of the above-described ALF fluorescent lamp which is obtained according to the present invention is 0.0012 W/lm. Using this ALF fluorescent lamp, the irradiance on the irradiated plane in the wavelength range of 600 nm to 1100 nm can be 0.1 W/$m^2$ or more by setting the illuminance to be 80 lx or more. When the illuminance is set to be 500 lx or more, effects similar to those obtained by using the above-described illumination light IL+TR can be expected. Moreover, the ALF fluorescent lamp does not have radiation at a wavelength equal to or greater than 1100 nm. Therefore, it is possible to prevent the thermal stress from adversely influencing the effect of maintaining/promoting the biofunctions provided by red to near infrared radiation.

Moreover, the ALF fluorescent lamp has a small radiation peak in the wavelength range of 700 nm to 900 nm, and has a radiant efficiency of 0.0011 W/lm in the wavelength range of 700 nm to 1100 nm. Light having a wavelength of 700 nm or more has a visibility which is lower than that of a visible range wavelength or will not be perceived as light. Therefore, by using the ALF fluorescent lamp, it is possible to obtain the above-described effect provided by non-visible light radiation. i.e., the biofunctions can be maintained/promoted without the user observing any unusual color.

The chromaticity of the color of light provided by the above-described ALF fluorescent lamp is (x,y) = (0.4485, 0.4172) , which is within ±0.01 from the blackbody radiation locus in the CIE 1960 UCS coordinate system. Therefore, the color of the light is white, and thus the illumination light will cause no discomfort and will not inhibit the effect of maintaining/promoting biofunctions provided by red to near infrared radiation.

For comparison purposes, Figure **7** shows the relative spectral radiant energy distributions for the MFG fluorescent lamp and the ALF fluorescent lamp shown in Figures **5** and **6**, respectively, along with the distributions for an LED having an emission peak wavelength of 660 nm and for a 60 W silica incandescent lamp which are shown in Figure **1**.

Moreover, Table 3 below shows, along with the data shown in Table 1 above, the illuminance (in lx) which is required for each of the above-described MFG fluorescent lamp and ALF fluorescent lamp so that the irradiance at a wavelength equal to or greater than 635 nm on the forehead of a subject is equivalent to that shown in Reference 1, i.e., the illuminance (in lx) which is required to obtain an NK cell activity equivalent to that obtained by using an LED in Reference 1. Specifically. Table 3 shows, as does Table 1, the irradiance for each light source at a wavelength in the range of 635 nm to 1000 nm and the illuminance which is required to obtain, with a 30 minute irradiation, an NK cell activity equivalent to that obtained by a 30 minute irradiation at an illuminance of 80 lx by using an LED having an emission peak wavelength of 660 nm. The 635 nm wavelength is a half-width wavelength of the LED having an omission peak wavelength of 660 nm.

Table 3

| Illuminance required for ALF and MFG fluorescent lamps to obtain NK activity equivalent to that obtained by using LED | | |
|---|---|---|
| | Irradiance (635-1000 nm) μW/$cm^2$/1000 lx | Illuminance required for the same irradiance as that obtained by using LED 80 lx |
| LED (660 nm) | 167.3 | 80 lx (30 min) |
| ALF fluorescent lamp | 26.0 | 515 lx (30 min) |
| MFG fluorescent lamp | 12.4 | 1079 lx (30 min) |
| Three-band type fluorescent lamp (daylight) | 5.2 | 2574 lx (30 min) |
| White light fluorescent lamp | 7.1 | 1885 lx (30 min) |
| Silica 60 W | 283.2 | 47 lx (30 min) |
| 635 nm is half-width wavelength of LED 660 nm | | |

Thus, it can be seen that an NK cell activity equivalent to that obtained in Reference 1 by using an LED can be obtained with an illuminance of about 1000 lx for the MFG fluorescent lamp and about 500 lx for the ALF fluorescent lamp.

When a general purpose lighting fixture having the above-described MFG fluorescent lamp or ALF fluorescent lamp attached thereto is provided in an office, for example, it is possible to provide a worker working under the illumination with the illuminance required for the work while providing the effect of maintaining/promoting biofunctions, e.g., activating the functions of the autonomic nervous system or improving the strength of the immune system, thereby eliminating the health condition concern of the worker who has only a little chance to be exposed to daylight.

(Second Embodiment)

Next, the second embodiment of the light (radiant energy) radiation apparatus of the present invention will be described, which is obtained by adding, to a general purpose lighting fixture, a radiation source for red to near infrared radiation including a discharge lamp or a light emitting diode in addition to a conventional fluorescent lamp.

Figure **8** schematically illustrates a configuration of a light radiation apparatus (lighting fixture) **1** according to the present embodiment. As shown in Figure **8**, the lighting fixture **1** of the present embodiment includes, in combination, a ring-shaped fluorescent lamp **2** as an illumination source, and a radiation source **3** for red to near infrared radiation (hereinafter, referred to as the "near infrared radiation source **3"**). The near infrared radiation source **3** may be, for example, an infrared light emitting diode (LED).

The wavelength range for red to near infrared radiation from the near infrared radiation source **3** is set to, as described above, 600 nm to 1100 nm in principle, more practically 635 nm to 1100 nm, and more preferably 700 nm to 1100 nm in order to pursue the effects provided by radiation of non-visible light.

As described above with reference to Figure **4**, the "organism window" exists in the wavelength range of 700 nm to 1100 nm, and light having a wavelength in this range will not be absorbed by water or hemoglobin existing in the organism but rather permeate into the organism efficiently. Particularly, for a wavelength range of 800 nm to 1000 nm in the organism window, the absorption by water and hemoglobin will be significantly reduced, thereby allowing for more effective permeation of light (radiant energy) into the organism.

Therefore, the center for controlling biofunctions in the vicinity of the hypothalamus can be more efficiently controlled by using a wavelength range which efficiently permeates into the organism, e.g., light having an emission peak wavelength of 880 nm which is obtained by a light emitting diode made of AlGaAs, instead of light having an emission peak wavelength of 660 nm used in the conventional configuration (Reference 1).

Herein, light at a wavelength of 880 nm is beyond the sensibility of the human eyes. Therefore, where infrared radiation light from a lighting fixture having this wavelength is localized to a position of a human, or where there is provided a mechanism for tracking a worker with this light, even if the irradiance variations change substantially, the variations and/or fluctuations in the irradiance will not be perceived by a human because a human will not recognize the radiated light itself. Therefore, where light in a visible wavelength range is radiated, variations and/or fluctuations in the irradiance of the radiated light may cause a human or worker (the organism irradiated) to feel uncomfortable. However, such a discomfort will not occur when using illumination light having such a wavelength.

Thus, by additionally using a light source in which the wavelength of emitted light is within the wavelength band such that the biofunctions can be maintained/promoted by non-visible light, it is possible to obtain a light radiation apparatus and method which does not adversely affect the light environment created by the illumination light and will not cause a problem visually.

The present inventors have experimentally confirmed that the parasympathetic nerve is better activated by using illumination light obtained by adding radiation from a light emitting diode having an emission peak wavelength of 880 nm to illumination light from a light bulb color fluorescent lamp, than by using illumination light made solely of the illumination light from the light bulb color fluorescent lamp. The results of the experiments will be discussed below.

In the experiments, one male subject who was in his forties was instructed to sit on a chair provided in a booth illuminated by illumination light from a light bulb color fluorescent lamp which provides an on-desk illuminance of 200 lx (hereinafter, referred to as "EX-L illumination light"), and assigned a 20-minute reading task in the booth. The illuminance on the forehead of the subject was 300 lx. The first ten minutes in this reading task were provided for the subject to get accustomed to the EX-L illumination light. During the latter ten minutes in the reading task, the electrocardiogram complex of the subject was measured.

After the 20-minute reading task under such EX-L illumination light, the subject was instructed to sit on a chair in a booth illuminated by illumination light obtained by adding light radiated from a light emitting diode (LED) made of AlGaAs and having an emission peak wavelength of 880 nm to illumination light from a light bulb color fluorescent lamp which provides an on-desk illuminance of 200 lx (hereinafter, referred to as "+880 nm illumination light"), and assigned a 20-minute reading task in the booth. The illuminance on the forehead of the subject was 300 lx, and the irradiance of radiation from the LED was set to be 1.2 $W/m^2$ on the forehead of the subject. As described above, since radiation at a wavelength of 880 nm is in a wavelength range such that it cannot be perceived by human eyes, there is no apparent difference between the EX-L illumination light and the +880 nm illumination light. Again, the first ten minutes in the reading task under the +880 nm illumination light were provided for the subject to get accustomed to the +880 nm illumination light. During the latter ten minutes in the reading task, the electrocardiogram complex of the subject was measured.

Based on the electrocardiogram complex measured as described above, changes in the heart beat of the subject were subjected to a frequency analysis. Among the frequency components of the changes in the heart beat, the high frequency range (HF) of 0.15 to 0.40 Hz reflects the activity of the parasympathetic nerve, and the lower frequency range (LF) of 0.04 to 0.15 Hz reflects the activity of both the sympathetic nerve and the parasympathetic nerve (see, for example, Hiroshi Hayashi ed., "Clinical Application of Changes in the Heart Beat - Physiological Significance. Disease Evaluation, Prognosis -", IGAKUSHOIN, 1999). This shows that when the ratio LF/HF between the integral, values of the respective frequency bands is large, the sympathetic nerve has a higher activity than the parasympathetic nerve, and when the value LF/HF is small, the parasympathetic nerve has a higher activity than the sympathetic nerve.

Based on the results obtained by repeating five times the heart beat measurement according to the above-described measurement procedure, the average value of LF/HF was calculated for each of the illumination light (EX-L illumination light and +880 nm illumination light), thereby obtaining a value 1.91 for the EX-L illumination light and 1.77 for the +880 nm illumination light. Thus, it was confirmed that it was possible to improve the activity of the parasympathetic nerve by using the +880 nm illumination light to which radiation at a wavelength of 880 nm, invisible to human eye, was added, than by using the EX-L illumination light made solely of the illumination light from the light bulb color fluorescent lamp.

(Third Embodiment)

Next, as a light (radiant energy) radiation apparatus according to the third embodiment of the present invention, a configuration of the lighting fixture **1** will now be described, which is obtained by adding, to a general purpose lighting fixture, the radiation source **3** for red or near infrared radiation such as an LED in addition to the illumination fluorescent lamp **2**, and in which the on/off control of the added radiation source **3** is performed independently of the control for the illumination fluorescent lamp **2**.

The configuration of the lighting fixture of the present embodiment is similar to that of the second embodiment shown in Figure **8** except that in the present embodiment, the on/off control of the ring-shaped fluorescent lamp **2** and the on/off control of the near infrared radiation source **3** can be performed independently. Thus, a person who lives or works under the illumination from the lighting fixture **1** of the present embodiment can control the on/off state of radiation so as to maintain/promote their biofunctions. For example, when the person stops working, the person can turn off only the illumination light source (ring-shaped fluorescent lamp) **2** so as to receive only infrared radiation from the near infrared radiation source **3** during the break.

As described above with reference to Figure **4**, the "organism window" exists in the wavelength range of 700 nm to 1100 nm, and light having a wavelength in this range will not be absorbed by water or hemoglobin existing in the organism but rather permeate into the organism efficiently. Particularly, for a wavelength range of 800 nm to 1000 nm in the organism window, the absorption by water and hemoglobin will be significantly reduced, thereby allowing for more effective permeation of light (radiant energy) into the organism. Therefore, the center for controlling biofunctions in the vicinity of the hypothalamus can be more efficiently controlled by using a light source capable of emitting light in a wavelength range which efficiently permeates into the organism, e.g., a light emitting diode made of AlGaAs which emits light having an emission peak wavelength of 880 nm, instead of an LED emitting light having an emission peak wavelength of 660 nm used in the conventional configuration (Reference 1), as the near infrared radiation source **3** in the lighting fixture **1** of the present embodiment.

Herein, light at a wavelength of 880 nm is beyond the sensibility of the human eyes. Therefore, where infrared radiation light from a lighting fixture having this wavelength is localized to a position of a human, or where there is provided a mechanism for tracking a worker with this light, even if the irradiance variations change substantially, the variations and/or fluctuations in the irradiance will not be perceived by a human because a human will not recognize the radiated light itself. Therefore, where light in a visible wavelength range is radiated, variation, and/or fluctuations in the irradiance of the radiated light may cause a human or worker (the organism irradiated) to feel uncomfortable. However, such a discomfort will not occur when using illumination light having such a wavelength.

In the present embodiment, the visible light source (illumination light source) **2** and the near infrared radiation source **3** can be controlled independently. Alternatively, at least one of the controls can be performed independently. For example, the illuminance for visually perceiving the object to be viewed and the irradiance of near infrared radiation for maintaining/promoting biofunctions can be modulated with one signal by dimming the output from the visible light source **2** and interlocking the output with the output from the near infrared radiation source **3**. Alternatively, the output of the visible light source **2** and the output of the near infrared radiation source **3** may be controlled to be inversely proportional to each other. In such a case, when the illuminance is high, the amount of energy consumed may be conserved by primarily using the energy for the visible emission which is required to perceived the object to be viewed during work. When the illuminance is low, it is possible to primarily provide near infrared radiation which is required for maintaining/promoting biofunctions so as to ease the person's mind after the work.

In a light illumination apparatus as described above including, in combination, a visible light source and near infrared radiation source, at least one of which can be controlled independently, a control signal for turning on/dimming the light sources can be generated directly or indirectly via an operation section from external information input means such as a switch, a dial, a button, and a keyboard. With such a configuration, the use or operator can suitably set not only the illuminance but also the near infrared radiation irradiance according to the circumstances.

In a light illumination apparatus as described above including, in combination, a visible light source and a near infrared radiation source, at least one of which can be controlled independently, a control signal for turning on/dimming the light sources can alternatively be output based on a predetermined program according to information in the illumination apparatus such as the time or the elapsed time after the light is turned on. With such a configuration used in an office, for example, it is possible to conserve the amount of energy consumed by increasing only the output from the visible light source during working hours, and to maintain/promote biofunctions of people in the office by lowering the output from the visible light source while increasing the output from the near infrared radiation source during breaks. Thus, it is possible to rest more effectively. For either house illumination or office illumination, in places which are inside a building but to which near infrared radiation included in direct/indirect light from the sun can reach, it is possible to output only visible light required for visually perceiving the object to be viewed during the daytime and to output near infrared radiation only at night.

In a light illumination apparatus as described above including, in combination, a visible light source and a near infrared radiation source, at least one of which can be controlled independently, the control signal for turning on/dimming the light sources may need to be generated based on both the external information and the internal information. This can be achieved by providing a determination section in which a program has previously been incorporated for determining whether the independent on/off control signal should be generated based on the signal from the external information input means or the signal from the internal information. For example, in places which are inside a building but to which near infrared radiation included in direct/indirect light from the sun can reach, there may be cases where it is desired to output near infrared radiation even when near infrared radiation should not be necessary (e.g., during the daytime) based on the internal information (time), e.g., when the window surface is covered with a shield such as a curtain or when it is desired to increase the potential effect of maintaining/promoting biofunctions. In such a case, near infrared radiation can be output by giving the control based on the external information (a signal from a switch, or the like) a higher priority.

The threshold value used to determine whether or not to generate a control signal for turning on/dimming the light source based on the internal information or the external information can be previously, i.e., during the manufacture, set by the manufacturer of the lighting fixture. Moreover, where the threshold value can be reset to a different value, the user can change the threshold value as necessary so as to configure the lighting fixture which can accommodate the user's convenience or any circumstances. The threshold value can be automatically changed based on learning by an algorithm, e.g., the fuzzy theory, using the history of the external information and the internal information. In such a case, it is possible to realize a convenient lighting fixture with which the user does not have to do any operation of changing the settings.

The configuration of the light (radiant energy) radiation apparatus according to the present invention which is capable of rendering the parasympathetic nerve dominant can be used, for example, for illumination in the bedroom. The apparatus can be used before a person in the bedroom goes to sleep when radiation includes visible light, and the apparatus can be used after the person goes to sleep when radiation includes only near infrared radiation. In either case, the use of the apparatus can reduce the heart rate by activating the parasympathetic nerve, thereby allowing the person to sleep well.

Alternatively, the configuration of the light (radiant energy) radiation apparatus according to the present invention which is capable of rendering the parasympathetic nerve dominant, can be used, for example, for illumination in the dining room. In such a case, it is possible to promote secretion of digestive fluid by activating the parasympathetic nerve, while illuminating food with visible light. Alternatively, the configuration of the light (radiant energy) radiation apparatus according to the present invention which is capable of rendering the parasympathetic nerve dominant, can be used for example, for illumination in the rest room. In such a case, it is possible to promote peristalsis of the intestines by activating the parasympathetic nerve, thereby allowing for smooth excretion, while providing visible light. Alternatively, the configuration of the light (radiant energy) radiation apparatus according to the present invention which is capable of rendering the parasympathetic nerve dominant, can be used for example, for illumination in the bathroom. In such a case, it is possible to suppress the heart rate by activating the parasympathetic nerve, thereby mentally and physically relaxing a person in the bathroom in addition to relaxation effects from bathing, while providing visible light.

Alternatively, the configuration of the light (radiant energy) radiation apparatus according to the present invention which is capable of rendering the sympathetic nerve dominant, can be used for example, for illumination in the kitchen. In such a case, it is possible to increase the vigilance of a person in the kitchen by activating the sympathetic nerve, thereby increasing the safety for the use of knives and fire, while providing visible light. Alternatively, the configuration of the light (radiant energy) radiation apparatus according to the present invention which is capable of rendering the sympathetic nerve dominant, can be used for example, for illumination of roads and streets. In such a case, it is possible to increase the vigilance of a person on a road or a street by activating the sympathetic nerve, thereby allowing the person to make quick determinations for avoiding dangers from obstacles while providing visible light. Alternatively, the configuration of the light radiation apparatus according to the present invention which is capable of rendering the sympathetic nerve dominant, can be incorporated for example, in the headrest of a driver's seat of a car. In such a case, it is possible to increase the vigilance of the driver by activating the sympathetic nerve, thereby allowing the driver to make quick determinations for avoiding dangers from obstacles. Alternatively, the configuration of the light radiation apparatus according to the present invention which is capable of rendering the sympathetic nerve dominant, can be used, for example, in an alarm clock. In such a case, it is possible to waken the user of the alarm clock quickly by activating the sympathetic nerve.

The light (radiant energy) radiation apparatus according to the present invention can be used in a discharge lamp or a fluorescent discharge lamp having radiant energy of a predetermined wavelength which provides desirable effects, e.g., those in a range of 600 nm (or 635 nm) to 1100 nm. Alternatively, the light (radiant energy) radiation apparatus may be provided in the form of a discharge lamp or a fluorescent discharge lamp in which radiant energy in the wavelength range of 600 nm (or 635 nm) to 1100 nm is equal to or greater than 15% of radiant energy in a visible wavelength range of 380 nm to 780 nm. With such a discharge lamp or a fluorescent discharge lamp having such features as described above, the characteristics of radiated light can be set so that the deviation (duv) of the chromaticity of light from a Planckian locus in CIE 1960 UCS chromaticity diagram is within ±0.01 in order to ensure that the color of light would not be uncomfortable, as described above. Alternatively, the characteristics of radiated light can be set so that: in the emission spectrum, radiant energy in a wavelength range of 700 nm to 1100 nm, which deeply and efficiently permeates into an organism thereby providing an effect of improving the strength of the immune system and/or activating the autonomic nervous system, is equal to or greater than 15% of radiant energy in a visible wavelength range of 380 nm to 780 nm; and the deviation (duv) of the chromaticity of light from a Planckian locus in CIE 1960 UCS chromaticity diagram is within ±0.01 in order to ensure that the color of light would not be uncomfortable.

In the light (radiant energy) radiation apparatus and method of the present invention, radiant energy in the wavelength range as described above in the foregoing embodiments may be radiated in the form of an alternating or pulsed wave with a frequency of 0.5 Hz to 13 Hz. In such a case, the configuration of the light (radiant energy) radiation apparatus may be similar to that described above in the third embodiment with reference to Figure **8**, for example.

When radiation in a near infrared wavelength range is provided by alternating or pulsed light, as described above, the center for controlling biofunctions in the vicinity of the hypothalamus can be more efficiently controlled by using a wavelength range which efficiently permeates into the organism. e.g., light having an emission peak wavelength of 880 nm which is obtained by a light emitting diode made of AlGaAs, instead of light having an emission peak wavelength of 660 nm used in the conventional configuration (Reference 1), in view of the presence of the "organism window" wavelength range described above with reference to Figure **4**. Since this wavelength range is beyond the sensibility of the human eyes, infrared radiation in this wavelength range will not cause flickering or a discomfort.

Radiation in the near infrared wavelength range can be provided in the form of alternating or pulsed light, as described above, with a discharge lamp or a fluorescent discharge lamp in which radiant energy of a predetermined wavelength which provides desirable effects, e.g., those in a range of 600 nm (or 635 nm) to 1100 nm, is equal to or greater than 15% of radiant energy in a visible wavelength range of 380 nm to 780 nm. With such a discharge lamp or a fluorescent discharge lamp having such features as described above, the characteristics of radiated light can be set so that the deviation (duv) of the chromaticity of light from a Planckian locus in CIE 1960 UCS chromaticity diagram is within ±0.01 in order to ensure that the color of light would not be uncomfortable, as described above.

Moreover, it is easy to turn on infrared radiation for maintaining/improving NK cell activity and/or biofunctions only during a predetermined or desired period of time by separately providing a light source for the visible wavelength range and a light source for the infrared range so that the respective on/off controls of the light sources are independently performed.

In the above description of the second and third embodiments of the present invention, with respect to the configuration in which the light source (radiation source) **2** for radiation in the visible wavelength range and the light source (radiation source) **3** for radiation in the red to near infrared region are separately provided, the two light sources (radiation sources) **2** and **3** are attached to the same body. However, the configuration of the present invention is not limited to such a particular configuration. Similar effects are obtained in cases as follows. For example, the light source (radiation source) **2** for radiation in the visible wavelength range may be provided on a wall surface in the room, while providing the light source (radiation source) **3** for radiation in the red to near infrared region on the opposing wall surface in the room. Alternatively, the light source (radiation source) **2** for radiation in the visible wavelength range may be provided on the ceiling near a wall surface in the room, while providing the light source (radiation source) **3** for radiation in the red to near infrared region on the ceiling near the opposing wall surface in the room.

(Fourth Embodiment)

The fourth embodiment of the present invention will now be described. In the fourth embodiment, the light (radiant energy) radiation apparatus of the present invention as described above in the foregoing embodiments is provided with an image display function. The fourth embodiment provides a display apparatus which is obtained by using the function of radiating light (radiant energy) according to the present invention.

The display apparatus applications to which the present invention can be applied include, for example, a computer display apparatus, a game display apparatus, or a TV image display apparatus, and the like. A light source (radiation source of radiant energy) for irradiating a viewer of the display screen (e.g., a person who does work with a computer while watching the display screen) with light (more generally, radiant energy) in the above-described red to near infrared wavelength range can be provided along the frame or around the display section of such apparatuses. In such a case, it is possible to expose the viewer watching the display screen to the red to near infrared radiation, thereby maintaining/promoting the biofunctions of the viewer.

More specific exemplary configurations of such apparatuses will be described below with reference to the accompanying drawings. Figure **9** shows a display apparatus 10 according to the present embodiment which includes an ordinary display section **11** and a red to infrared radiation source **12** (referred to as the "near infrared radiation source **12"**), e.g., a light emitting diode (LED), or the like, mounted on top of the ordinary display section **11**. Again, the center for controlling biofunctions in the vicinity of the hypothalamus can be more efficiently controlled by using an LED emitting light in a wavelength range which efficiently permeates into the organism, e.g., an LED made of AlGaAs, instead of an LED emitting light having an emission peak wavelength of 660 nm used in the conventional configuration (Reference 1), as the near infrared radiation source **12**. Since this wavelength range is beyond the sensibility of the human eyes, near infrared radiation in this wavelength range will not be a source of an uncomfortable glare, and will not cause flickering or a discomfort.

Alternatively, the MFG fluorescent lamp described above in the first embodiment or the ALF fluorescent lamp described above in the second embodiment can be used as the above-described near infrared radiation source **12**. For example, the ALF fluorescent lamp; whose main emission wavelength range is 700 nm to 800 nm, can be used to replace the above-described AlGaAs LED.

The near infrared radiation source **12** may be configured so that the near infrared radiation source **12** can be turned on independently of the display section **11**, and the user can turn on the near infrared radiation source **12** when necessary.

Next, Figure **10** shows another display apparatus **20** of the present embodiment which has a different configuration. The display apparatus **20** includes an ordinary display section **21** and a near infrared radiation source **22**, e.g., an LED, provided on top of the ordinary display section **21**. The display apparatus **20** further includes an infrared sensor **23** for detecting radiation from the near infrared radiation source **22** which is reflected by a viewer **24** who watches the display or works with the display. Based on the outputs from the infrared sensor **23**, the irradiance of infrared radiation on the viewer **24** is obtained. The radiation output power of the near infrared radiation source **22** can be adjusted based on the obtained irradiance. Thus, the display apparatus **20** of Figure **10** has a function of constantly maintaining the infrared radiation level to which the viewer **24** is exposed.

Generally, for infrared radiation in the wavelength range of 700 nm to 1100 nm, the face, which is not covered by hair or clothing, will have a relatively high reflectance. It can be made easier to control the amount of infrared radiation onto the viewer **24** by limiting the spectral sensitivity of the infrared sensor **23** to a wavelength range in the vicinity of the main wavelength of the near infrared radiation source **22** (e.g., limiting the spectral sensitivity of the infrared sensor **23** to a wavelength range in the vicinity of 880 nm when an AlGaAs LED having a peak wavelength of 880 nm is used as the near infrared radiation source **22**).

Moreover, a two-dimensional imaging device such as a CCD can be used as the infrared sensor **23** to image an area in front of the display section **21**. In such a case, the area of the face of the viewer **24** can be determined as an area where the brightness of reflected infrared radiation is greatest so as to obtain the irradiance of infrared radiation on the face of the viewer **24** based on the brightness of that area. In this way, it is possible to more precisely irradiate the face of the viewer **24** with a constant amount of infrared radiation.

Reference **3** reports that where the forehead of a user is irradiated with light from a red LED whose wavelength is 660 nm, it is possible to increase NK cell activity by using pulsed light at 0.5 to 13 Hz as the light with which the user is irradiated, rather than when irradiating the user with continuous constant light which is not modulated. In this regard, it is expected that it is possible to maintain/promote biofunctions by alternating or pulse-modulating at 0.5 to 13 Hz radiation in an infrared wavelength region which corresponds to the "organism window" (which more efficiently permeates into an organism to stimulate the hypothalamus in the brain) as described above with reference to Figure **4**.

While flashing light in the visible wavelength range around 10 Hz may cause epileptic seizure, a wavelength range corresponding to the "organism window" as described above is beyond the sensibility of the human eyes, and thus will not cause discomfort even when flashed within the field of view of the viewer **24**.

Various display apparatus applications will now be described to which the light (radiant energy) radiation method and apparatus of the present invention can be applied.

In the following description of the various displays, it will be assumed that the radiation wavelength range which provides the effect of maintaining/promoting biofunctions to be 600 nm to 1100 nm. Different values may be required for the lower limit of this range depending upon the contents to be displayed on the display and/or the environment in which the display is to be used.

For example, a color display apparatus may use red light of a wavelength in the vicinity of 635 nm. Therefore, the lower limit of the above-described wavelength range should be 635 nm or more where radiant energy for maintaining/promoting biofunctions is radiated independently of the displayed image. In order to radiate radiant energy for maintaining/promoting biofunctions without influencing the image or the color of the surrounding environment, the lower limit of the wavelength range is set to 700 nm or more for which the sensitivity of the human eyes (visibility) is low.

A CRT display produces an image made of pixels from emission from a fluorescent substance. A fluorescent plane can be provided by using a fluorescent substance as the fluorescent substance of the CRT display, which has a main emission section in the visible wavelength range and a sub emission section in a wavelength range of 600 nm to 1100 nm so as to radiate radiant energy in a wavelength range which permeates into an organism; thereby maintaining/promoting biofunctions of the user of an irradiated plane.

Alternatively, a fluorescent plane can be provided by using, as the fluorescent substance of the CRT display, a fluorescent substance which is obtained by mixing together a fluorescent substance material which emits visible light and another fluorescent substance material whose emission spectrum is in the wavelength range of 600 nm to 1100 nm so as to radiate radiant energy in a wavelength range which permeates into an organism, thereby maintaining/promoting biofunctions of the user of an irradiated plane.

Alternatively, in a CRT display, an emission section for producing an image made of pixels by emission from a fluorescent substance in a visible wavelength range and a fluorescent plane for radiating radiant energy in the wavelength range of 600 nm to 1100 nm can be overlapped with each other or adjacent to each other, and a part of driving energy to be supplied to the emission section for emitting light in the visible wavelength range can be supplied to the fluorescent plane for radiating radiant energy in the wavelength range of 600 nm to 1100 nm so as to radiate radiant energy in a wavelength range which permeates into an organism, thereby maintaining/promoting biofunctions of the user of an irradiated plane. Such a configuration can be realized by, for example, applying a fluorescent substance which radiates radiant energy in the wavelength range of 600 nm to 1100 nm around each of the fluorescent dots forming the emission section. In such a case, some of the electron beams with which the fluorescent dots are irradiated reach the fluorescent substance which radiates radiant energy in the wavelength range of 600 nm to 1100 nm due to scattering or displacement from an aimed position, thereby radiating radiant energy in the wavelength range of 600 nm to 1100 nm.

Alternatively, in a CRT display, an emission section for producing an image made of pixels by emission from a fluorescent substance in a visible wavelength range and a fluorescent plane for radiating radiant energy in the wavelength range of 600 nm to 1100 nm can be provided, and driving energy can be supplied to the fluorescent plane for radiating radiant energy in the wavelength range of 600 nm to 1100 nm independently of the intensity of driving energy to be supplied to the emission section for emitting light in the visible wavelength range so as to radiate radiant energy in a wavelength range which permeates into an organism, thereby maintaining/promoting biofunctions of the user of an irradiated plane. Such a configuration can be obtained by, for example, providing a radiation source on an end surface of a phase plate for radiating radiant energy in the wavelength range of 600 nm to 1100 nm toward the center of the phase plate. In such a case, radiant energy in the wavelength range of 600 nm to 1100 nm extends across the entire surface of the phase plate while it is repeatedly reflected between the outer surface and the inner surface of the phase plate. In such a case, it is possible to radiate radiant energy in the wavelength range of 600 nm to 1100 nm toward the viewer of the CRT display with the phase plate serving as a secondary radiation source.

A plasma display produces an image made of pixels from emission from a fluorescent substance. A fluorescent plane can be provided by using a fluorescent substance, as the fluorescent substance of the plasma display, which has a main emission section in the visible wavelength range and a sub emission section in a wavelength range of 600 nm to 1100 nm so as to radiate radiant energy in a wavelength range which permeates into an organism, thereby maintaining/promoting biofunctions of the user of an irradiated plane.

Alternatively, a fluorescent plane can be provided by using, as the fluorescent substance of the plasma display, a fluorescent substance which is obtained by mixing together a fluorescent substance material, which emits visible light and another fluorescent substance material whose emission spectrum is in the wavelength range of 600 nm to 1100 nm so as to radiate radiant energy in a wavelength range which permeates into an organism, thereby maintaining/promoting biofunctions of the user of an irradiated plane.

Alternatively, in a plasma display, an emission section for producing an image made of pixels by emission from a fluorescent substance in a visible wavelength range and a fluorescent plane for radiating radiant energy in the wavelength range of 600 nm to 1100 nm can be overlapped with each other or adjacent to each other, and a part of driving energy to be supplied to the emission section for emitting light in the visible wavelength range can be supplied to the fluorescent plane for radiating radiant energy in the wavelength range of 600 nm to 1100 nm so as to radiate radiant energy in a wavelength range which permeates into an organism, thereby maintaining/promoting biofunctions of the user of an irradiated plane. Such a configuration can be realized by, for example, applying a fluorescent substance which radiates radiant energy in the wavelength range of 600 nm to 1100 nm around each of the fluorescent dots forming the emission section. In such a case, some of the electron beams with which the fluorescent dots are irradiated reach the fluorescent substance which radiates radiant energy in the wavelength range of 600 nm to 1100 nm due to scattering or displacement from an aimed position, thereby radiating radiant energy in the wavelength range of 600 nm to 1100 nm.

Alternatively, in a plasma display, an emission section for producing an image made of pixels by emission from a fluorescent substance in a visible wavelength range and a fluorescent plane for radiating radiant energy in the wavelength range of 600 nm to 1100 nm can be provided, and driving energy can be supplied to the fluorescent plane for radiating radiant energy in the wavelength range of 600 nm to 1100 nm independently of the intensity of driving energy to be supplied to the emission section for emitting light in the visible wavelength range so as to radiate radiant energy in a wavelength range which permeates into an organism, thereby maintaining/promoting biofunctions of the user of an irradiated plane. Such a configuration can be obtained by, for example, providing a radiation source on an end surface of a phase plate for radiating radiant energy in the wavelength range of 600 nm to 1100 nm toward the center of the phase plate. In such a case, radiant energy in the wavelength range of 600 nm to 1100 nm extends across the entire surface of the phase plate while it is repeatedly reflected between the outer surface and the inner surface of the phase plate. In such a case, it is possible to radiate radiant energy in the wavelength range of 600 nm to 1100 nm toward the viewer of the plasma display with the phase plate serving as a secondary radiation source.

An electroluminescence (EL) display produces an image made of pixels from emission from a fluorescent substance. A fluorescent plane can be provided by using a fluorescent substance, as the fluorescent substance of the EL display, which has a main emission section in the visible wavelength range and a sub emission section in a wavelength range of 600 nm to 1100 nm so as to radiate radiant energy in a wavelength range which permeates into an organism. thereby maintaining/promoting biofunctions of the user of an irradiated plane.

Alternatively, a fluorescent plane can be provided by using, as the fluorescent substance of the EL display, a fluorescent substance which is obtained by mixing together a fluorescent substance material which emits visible light and another fluorescent substance material whose emission spectrum is in the wavelength range of 600 nm to 1100 nm so as to radiate radiant energy in a wavelength range which permeates into an organism, thereby maintaining/promoting biofunctions of the user of an irradiated plane.

Alternatively, in an EL display, an emission section for producing an image made of pixels by emission from a fluorescent substance in a visible wavelength range and a fluorescent plane for radiating radiant energy in the wavelength range of 600 nm to 1100 nm can be overlapped with each other or adjacent to each other, and a part of driving energy to be supplied to the emission section for emitting light in the visible wavelength range can be supplied to the fluorescent plane for radiating radiant energy in the wavelength range of 600 nm to 1100 nm so as to radiate radiant energy in a wavelength range which permeates into an organism, thereby maintaining/promoting biofunctions of the user of an irradiated plane. Such a configuration can be realized by, for example, applying a fluorescent substance which radiates radiant energy in the wavelength range of 600 nm to 1100 nm around each of the fluorescent dots forming the emission section. In such a case, some of the electron beams with which the fluorescent dots are irradiated reach the fluorescent substance which radiates radiant energy in the wavelength range of 600 nm to 1100 nm due to scattering, thereby radiating radiant energy in the wavelength range of 600 nm to 1100 nm.

Alternatively, in an EL display, an emission section for producing an image made of pixels by emission from a fluorescent substance in a visible wavelength range and a fluorescent plane for radiating radiant energy in the wavelength range of 600 nm to 1100 nm can be provided, and driving energy can be supplied to the fluorescent plans for radiating radiant energy in the wavelength range of 600 nm to 1100 nm independently of the intensity of driving energy to be supplied to the emission section for emitting light in the visible wavelength range so as to radiate radiant energy in a wavelength range which permeates into an organism, thereby maintaining/promoting biofunctions of the user of an irradiated plane. Such a configuration can be obtained by, for example, providing a radiation source on an end surface of a phase plate so as to radiate radiant energy in the wavelength range of 600 nm to 1100 nm toward the center of the phase plate. In such a case, radiant energy in the wavelength range of 600 nm to 1100 nm extends across the entire surface of the phase plate while it is repeatedly reflected between the outer surface and the inner surface of the phase plate. In such a case, it is possible to radiate radiant energy in the wavelength range of 600 nm to 1100 nm toward the viewer of the EL display with the phase plate serving as a secondary radiation source.

A light emitting diode (LED) display produces an image from an array of semiconductor devices each of which emits light in response to an input electric current. Such a display can be configured with LEDs whose emission spectrum includes a main emission section in the visible wavelength range and a sub emission section in a wavelength range of 600 nm to 1100 nm so as to radiate radiant energy in a wavelength range which permeates into an organism, thereby maintaining/promoting biofunctions of the user of an irradiated plane.

Alternatively, an LED display can be configured with LED devices obtained by integrating together LEDs whose emission spectrum is in the visible wavelength range and LEDs whose emission spectrum is in the wavelength range of 600 nm to 1100 nm so as to radiate radiant energy in a wavelength range which permeates into an organism, thereby maintaining/promoting biofunctions of the user of an irradiated plane.

Alternatively, in an LED display, an emission section for producing an image made of pixels by emission in a visible wavelength range and a fluorescent plane for radiating radiant energy in the wavelength range of 600 nm to 1100 nm can be overlapped with each other or adjacent to each other, and a part of driving energy to be supplied to the emission section can be supplied to the fluorescent plane so as to radiate radiant energy in a wavelength range which permeates into an organism, thereby maintaining/promoting biofunctions of the user of an irradiated plane. Such a configuration can be realized by for example, arranging LEDs which radiates radiant energy in the wavelength range of 600 nm to 1100 nm around the LEDs which emit light in the visible wavelength range. The LEDs can be connected in series or in parallel with each other, and driven by a control current corresponding to a video signal.

Alternatively, in an LED display, an emission section for producing an image made of pixels by emission in a visible wavelength range and a radiation section for radiating radiant energy in the wavelength range of 600 nm to 1100 nm can be provided, and driving energy can be supplied to the radiation section for radiating radiant energy in the wavelength range of 600 nm to 1100 nm independently of the intensity of driving energy to be supplied to the emission section so as to radiate radiant energy in a wavelength range which permeates into an organism, thereby maintaining/promoting biofunctions of the user of an irradiated plane. Such a configuration can be obtained by, for example, arranging LEDs which radiates radiant energy in the wavelength range of 600 nm to 1100 nm around the LEDs which emit light in the visible wavelength range, and applying a control current corresponding to a video signal to the LEDs which emit light in the visible wavelength range while applying a current independent of the control current to the LEDs which radiate radiant energy in the wavelength range of 600 nm to 1100 nm.

In a spontaneous emission liquid crystal display, an emission section for producing an image made of pixels by emission from a fluorescent substance in a visible wavelength, range and a fluorescent plane for radiating radiant energy in the wavelength range of 600 nm to 1100 nm can be provided, and driving energy can be supplied to the fluorescent plane for radiating radiant energy in the wavelength range of 600 nm to 1100 nm independently of the intensity of driving energy to be supplied to the emission section which emits light in the visible wavelength range so as to radiate radiant energy in a wavelength range which permeates into an organism, thereby maintaining/promoting biofunctions of the user of an irradiated plane. Such a configuration can be obtained by, for example, arranging a radiation source on an end surface of a liquid crystal panel so as to radiate radiant energy in the wavelength range of 600 nm to 1100 nm toward the center of the liquid crystal panel. In such a case, radiant energy in the wavelength range of 600 nm to 1100 nm extends across the entire liquid crystal panel while it is repeatedly reflected between the outer surface and the inner surface of the liquid crystal panel. In such a case, it is possible to radiate radiant energy in the wavelength range of 600 nm to 1100 nm toward the viewer of the liquid crystal display with the liquid crystal panel serving as a secondary radiation source.

A spontaneous emission liquid crystal display produces an image made of pixels by splitting and modulating a spatial intensity of light in a visible wavelength range. Such a display can be provided by using a light source including radiant energy in the visible wavelength range and the wavelength range of 600 nm to 1100 nm. Radiant energy in the wavelength range of 600 nm to 1100 nm can be superimposed on light in the visible wavelength range which has been split and modulated for producing the image made of pixels, or transmitted through the periphery of each pixel, so as to radiate radiant energy in a wavelength range which permeates into an organism, thereby maintaining/promoting biofunctions of the user of an irradiated plane.

A polarization film used in a liquid crystal device of such a spontaneous emission liquid crystal display is predisposed to impart a polarization effect on light in the visible wavelength range and no polarization effect on infrared radiation having a wavelength of at least 700 nm or more. Therefore, when a light source whose emission spectrum includes radiant energy in a wavelength range of 600 nm (or 635 nm) to 1100 nm in addition to the visible wavelength range is used as a back light, it is possible to irradiate the user with radiation in the wavelength range of 600 nm (or 635 nm) to 1100 nm with an intensity equal to or greater than a certain intensity irrespective of the type of image presented by spatially modulating an intensity distribution of visible light with liquid crystal.

In a projection liquid crystal display, an emission section for producing an image made of pixels by emission from a fluorescent substance in a visible wavelength range and a fluorescent plane for radiating radiant energy in the wavelength range of 600 nm to 1100 nm can be provided, and driving energy can be supplied to the fluorescent plane for radiating radiant energy in the wavelength range of 600 nm to 1100 nm independently of the intensity of driving energy to be supplied to the emission section which emits light in the visible wavelength range so as to radiate radiant energy in a wavelength range which permeates into an organism, thereby maintaining/promoting biofunctions of the user of an irradiated plane. Such a configuration can be obtained by, for example, arranging a radiation source on an end surface of a liquid crystal panel so as to radiate radiant energy in the wavelength range of 600 nm to 1100 nm toward the center of the liquid crystal panel. In such a case, radiant energy in the wavelength range of 600 nm to 1100 nm extends across the entire liquid crystal panel while it is repeatedly reflected between the outer surface and the inner surface of the liquid crystal panel. In such a case, it is possible to radiate radiant energy in the wavelength range of 600 nm to 1100 nm toward the viewer of the liquid crystal display with the liquid crystal panel serving as a secondary radiation source.

A projection liquid crystal display produces an image made of pixels by splitting and modulating a spatial intensity of light in a visible wavelength range. Such a display can be provided by using a light source including radiant energy in the visible wavelength range and the wavelength range of 600 nm to 1100 nm. Radiant energy in the wavelength range of 600 nm to 1100 nm can be superimposed on light in the visible wavelength range which has been split and modulated for producing the image made of pixels, or transmitted through the periphery of each pixel, so as to radiate radiant energy in a wavelength range which permeates into an organism, thereby maintaining/promoting biofunctions of the user of an irradiated plane.

A polarization film used in a liquid crystal device of such a projection liquid crystal display is predisposed to impart a polarization effect on light in the visible wavelength range and no polarization effect on infrared radiation having a wavelength of at least 700 nm or more. Therefore, when a light source including radiant energy in a wavelength range of 600 nm (or 635 nm) to 1100 nm in addition to the wavelength range in the visible wavelength range emission spectrum is used as a back light, it is possible to irradiate the user with radiation in the wavelength range of 600 nm (or 635 nm) to 1100 nm with an intensity equal to or greater than a certain intensity irrespective of the type of image presented by spatially modulating an intensity distribution of visible light with liquid crystal.

As described above, the present invention can be used in various types of display apparatuses to provide a display apparatus such as a computer image display apparatus, a game image display apparatus, and a television (TV) image display apparatus. Such a display apparatus is provided with a radiation source which irradiates the viewer of the computer image (in the case of a computer image display apparatus), the player of the game (in the case of a game image display apparatus), and the viewer of video images (in the case of a television image reproduction/display apparatus), with radiant energy in the wavelength range of 600 nm (or 635 nm) to 1100 nm, so as to irradiate the viewer or the player with radiant energy in a wavelength range which permeates into an organism while the viewer or the player is watching the screen (a computer image, a game image, or a video image), thereby maintaining/promoting biofunctions of the viewer or the player.

A display apparatus such as a computer image display apparatus, a game image display apparatus, or a television image reproduction/display apparatus, can be configured to provide the viewer with a break period for maintaining/promoting biofunctions during an image pause period by changing the intensity of radiant energy in the wavelength range of 600 nm (or 635 nm) to 1100 nm if the image on the display section is not switched to another for a predetermined period of time. In such a case, a screen saver function can be obtained by automatically changing the image.

In such a display apparatus which provides the viewer with a break period for maintaining/promoting biofunctions during an image pause period, after the intensity of radiant energy in the wavelength range of 600 nm (or 635 nm) to 1100 nm is changed, if the image on the display section is not switched to another for a predetermined period of time, and when the image is switched to another thereafter, the intensity of radiant energy in this wavelength range can be returned to the previous intensity so as to regain the original state.

## INDUSTRIAL APPLICABILITY

As described above, the present invention employs light (radiant energy) radiation (particularly, radiation in red to near infrared region), whereby it is possible to provide an organism in daily life with radiation in a wavelength range capable of maintaining/improving biofunctions, e.g., improving the strength of the immune system and activating the functions of the autonomic nervous system, or to maintain/improve the NK cell activity.

Particularly, the apparatus for radiating light (radiant energy) of the present invention can be configured as a general purpose illumination apparatus. By providing an illumination apparatus having such a configuration, it is possible to, for example, maintain/improve biofunctions or the NK cell, activity of a person who inevitably lives or works under an artificial illumination over a long period of time and thus cannot be sufficiently exposed to daylight, or to provide a person always with a sufficient amount of radiation irrespective of the weather, the regionality, the season of the year, etc.

Alternatively, the light (radiant energy) radiation apparatus of the present invention can be provided with an image display function (display function) so that it functions as a display apparatus. For example, the light source (light radiation apparatus) of the present invention can be arranged around a screen of a display apparatus such as a TV or a computer display apparatus, so that it is possible to efficiently irradiate the face of the viewer or the worker with light in a red to near infrared region when they have their face in the vicinity of the screen as they watch TV or do work with the display. Thus, it is possible to maintain/improve biofunctions or the NK cell activity in the body of a person who watches a display over a long period of time, e.g., a person who watches TV or who does OA (Office Automation) work.

As described above, according to the present invention, it is possible to realize various effect such as irradiating an organism unobtrusively in daily life with light (radiant energy) so as to maintain/promote biofunctions and/or the NK cell activity.

**Claims**

1. A radiant energy radiation apparatus, comprising means for radiating illumination light for an illumination purpose, the illumination light containing radiation in a visible wavelength range and radiation in a predetermined wavelength range which permeates into an organism to maintain/promote biofunctions.

2. A radiant energy radiation apparatus according to claim 1, wherein the predetermined wavelength range is a range of 600 nm to 1100 nm.

3. A radiant energy radiation apparatus according to claim 1, wherein the predetermined wavelength range maintains/promotes the biofunctions by increasing a strength of an immune system of the organism.

4. A radiant energy radiation apparatus according to claim 1, wherein radiation in the predetermined wavelength range maintains/promotes the biofunctions by activating an autonomic nervous system.

5. A radiant energy radiation apparatus according to any one of claims 1 to 4, wherein radiation means for radiation in a visible wavelength range and radiation means for radiation in the predetermined wavelength range are integrated together.

6. A radiant energy radiation apparatus according to any one of claims 1 to 4, wherein radiation means for radiation in a visible wavelength range and radiation means for radiation in the predetermined wavelength range are independently provided from each other.

7. A radiant energy radiation apparatus according to any one of claims 1 to 6, wherein on an irradiated plane to be irradiated with the illumination light, an irradiance at a wavelength in a range of 600 nm to 1100 nm is 0.1 $W/m^2$ or more.

8. A radiant energy radiation apparatus according to any one of claims 1 to 6, wherein: radiation in the predetermined wavelength range is radiation in a range of 600 nm to 1100 nm; and the radiation in the range of 600 nm to 1100 nm is radiated while being pulse-modulated at 0.5 to 13 Hz.

9. A radiant energy radiation apparatus according to any one of claims 1 to 6, wherein on an irradiated plans to be irradiated with the illumination light, radiant energy of radiation at a wavelength in a range of 600 nm to 1100 nm is equal to or greater than 15% of radiant energy of radiation at a wavelength in a visible wavelength range of 380 nm to 780 nm.

10. A radiant energy radiation apparatus according to any one of claims 1 to 6, wherein a radiant efficiency of radiation at a wavelength in a range of 600 nm to 1100 nm is equal to or greater than 0.001 W/lm.

11. A radiant energy radiation apparatus according to any one of claims 1 to 6, wherein on an irradiated plane to be irradiated with the illumination light, radiant energy of radiation at a wavelength in a range of 1100 nm to 2.5 μm is smaller than radiant energy of radiation at a wavelength in a range of 600 nm to 1100 nm.

12. A radiant energy radiation apparatus according to any one of claims 1 to 6, wherein: the illumination light has a color of light which does not cause discomfort; and a deviation (duv) of the chromaticity of light from a Planckian locus in Commission Internationals de l'Eclairage (CIE) 1960 UCS chromaticity diagram is within ±0.01.

13. A radiant energy radiation apparatus according to any one of claims 9 to 12, wherein the apparatus has a configuration of a discharge lamp.

14. A radiant energy radiation apparatus according claim 13, wherein the apparatus has a configuration of a fluorescent discharge lamp.

**15.** A radiant energy radiation apparatus according to any one of claims 9 to 12, wherein the apparatus has a configuration of an incandescent lamp.

**16.** A radiant energy radiation apparatus according to any one of claims 9 to 12, wherein the apparatus has a configuration of a light source including a solid light emitting device.

**17.** A radiant energy radiation apparatus, comprising means for radiating radiation in a predetermined wavelength range which has a low visibility for a human and which deeply permeates into an organism to maintain/promote biofunctions.

**18.** A radiant energy radiation apparatus according to claim 17, wherein the predetermined wavelength range is a range of 600 nm to 1100 nm.

**19.** A radiant energy radiation apparatus according to claim 17, wherein radiation in the predetermined wavelength range maintains/promotes the biofunctions by increasing a strength of an immune system of the organism.

**20.** A radiant energy radiation apparatus according to claim 17, wherein radiation in the predetermined wavelength range maintains/promotes the biofunctions by activating an autonomic nervous system.

**21.** A radiant energy radiation apparatus according to any one of claims 17 to 20, wherein on an irradiated plane to be irradiated with radiation, an irradiance at a wavelength in a range of 700 nm to 1100 nm is 0.03 $W/m^2$ or more.

**22.** A radiant energy radiation apparatus according to any one of claims 17 to 20, wherein: radiation in the predetermined wavelength range is radiation in a range of 700 nm to 1100 nm; and radiation in the range of 700 nm to 1100 nm is radiated while being pulse-modulated at 0.5 to 13 Hz.

**23.** A radiant energy radiation apparatus according to any one of claims 17 to 20, wherein on an irradiated plane to be irradiated with radiation, radiant energy of radiation at a wavelength in a range of 1100 nm to 2.5 μm is smaller than radiant energy of radiation at a wavelength in a range of 700 nm to 1100 nm.

**24.** A radiant energy radiation apparatus according to any one of claims 21 to 23, wherein the apparatus has a configuration of a discharge lamp.

**25.** A radiant energy radiation apparatus according claim 24, wherein the apparatus has a configuration of a fluorescent discharge lamp.

**26.** A radiant energy radiation apparatus according to any one of claims 21 to 23, wherein the apparatus has a configuration of an incandescent lamp.

**27.** A radiant energy radiation apparatus according to any one of claims 21 to 23, wherein the apparatus has a configuration of a light source including a solid light emitting device.

**28.** A radiant energy radiation apparatus according to any one of claims 17 to 27, wherein the apparatus has an illumination function of providing illumination light for an illumination purpose.

**29.** A radiant energy radiation apparatus according to any one of claims 17 to 27, wherein the apparatus has a display function of displaying a predetermined image.

**30.** A radiant energy radiation apparatus according to claim 29, wherein the predetermined image is displayed by the means for radiating radiation in the predetermined wavelength range.

**31.** A radiant energy radiation apparatus according to claim 29, further comprising display means for displaying the predetermined image, wherein the means for radiating radiation in the predetermined wavelength range is attached to the display means.

*FIG. 1*

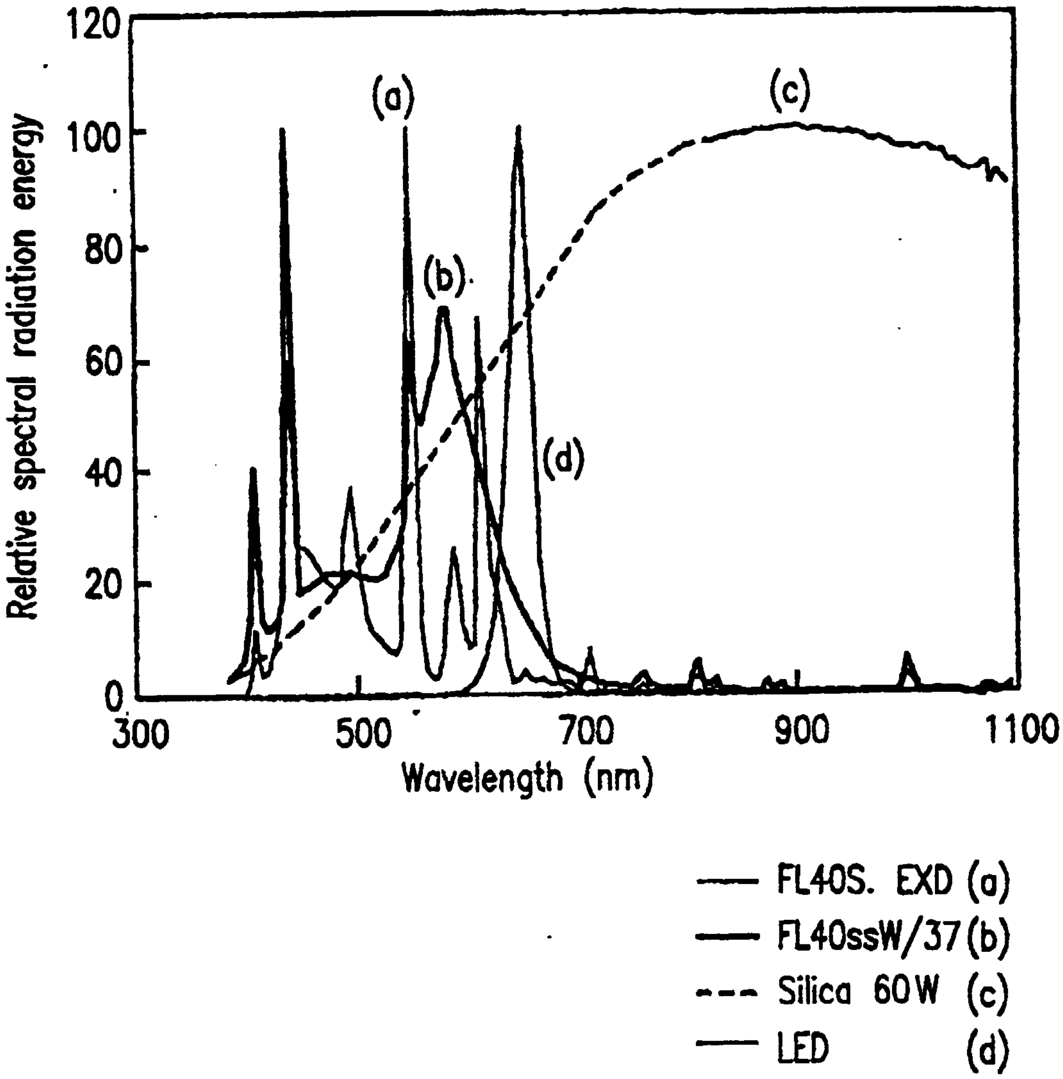
120
100
80
60
40
20
0
Relative spectral radiation energy
300
500
700
900
1100
Wavelength (nm)
(a)
(b)
(c)
(d)
FL40S. EXD (a)
FL40ssW/37 (b)
Silica 60W (c)
LED (d)

FIG.2

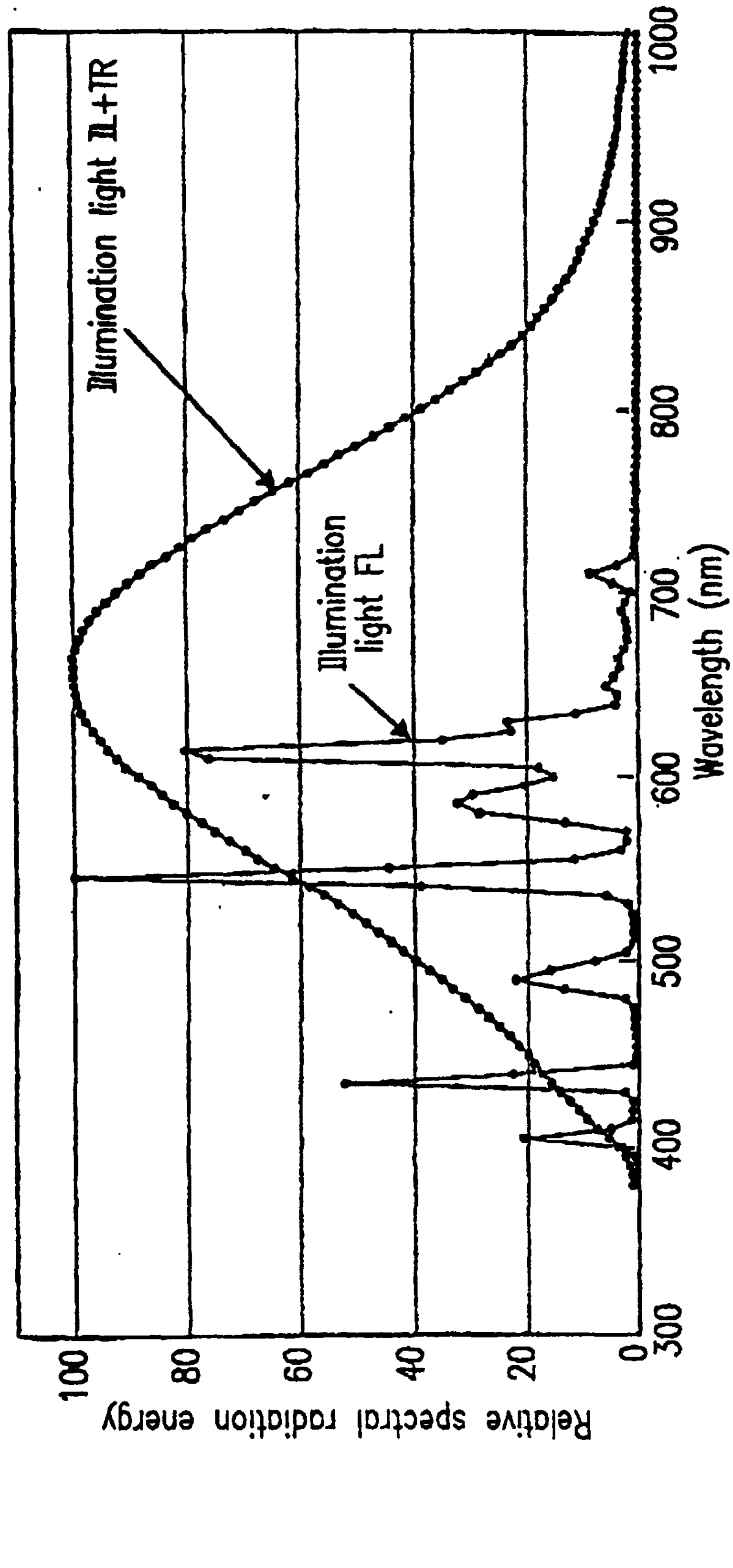
Relative spectral radiation energy
100
80
60
40
20
0
300
400
500
600
700
800
900
1000
Wavelength (nm)
Illumination light IL+TR
Illumination light FL

FIG.3

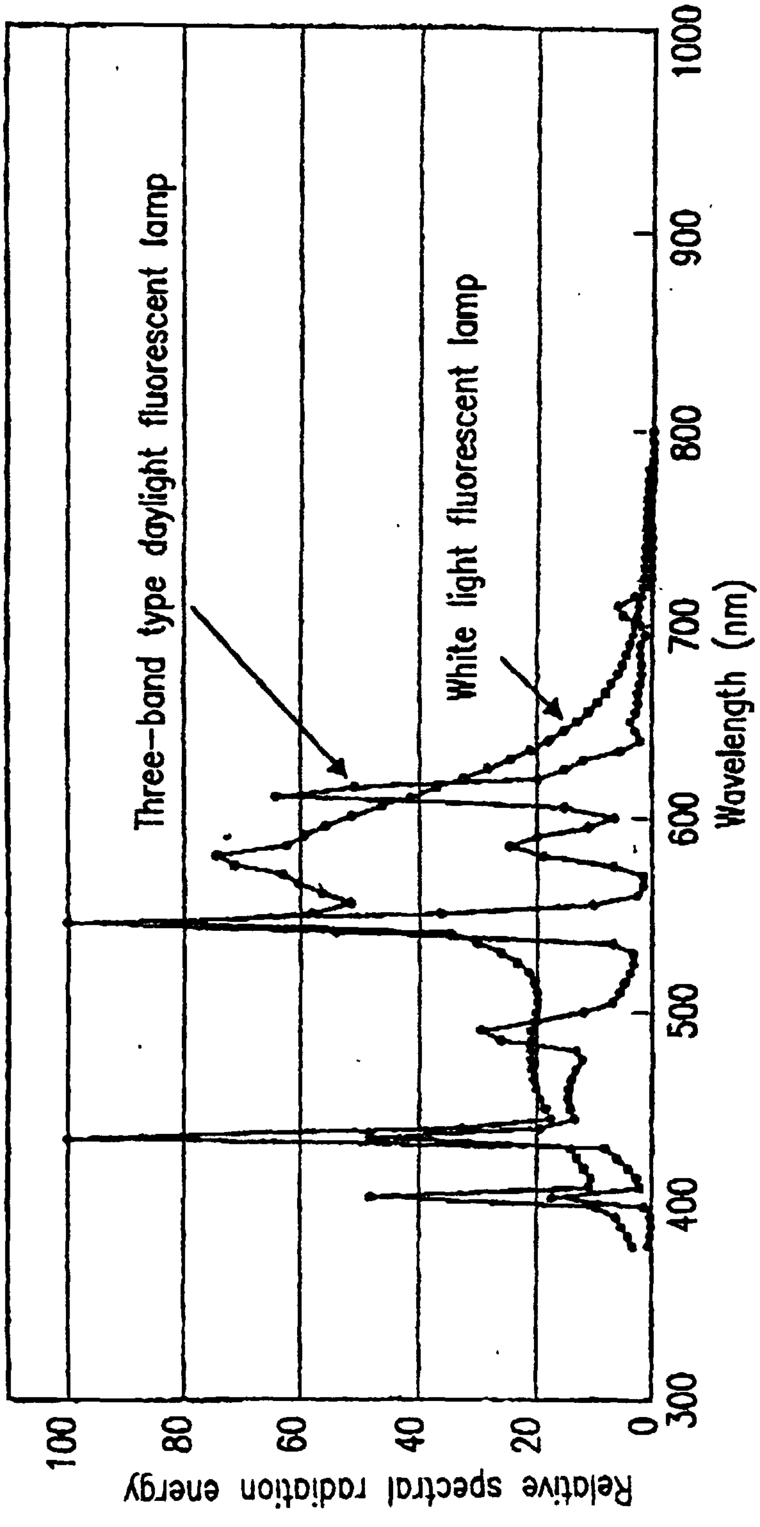
Three-band type daylight fluorescent lamp
White light fluorescent lamp
Relative spectral radiation energy
0
20
40
60
80
100
Wavelength (nm)
300
400
500
600
700
800
900
1000

*FIG. 4*

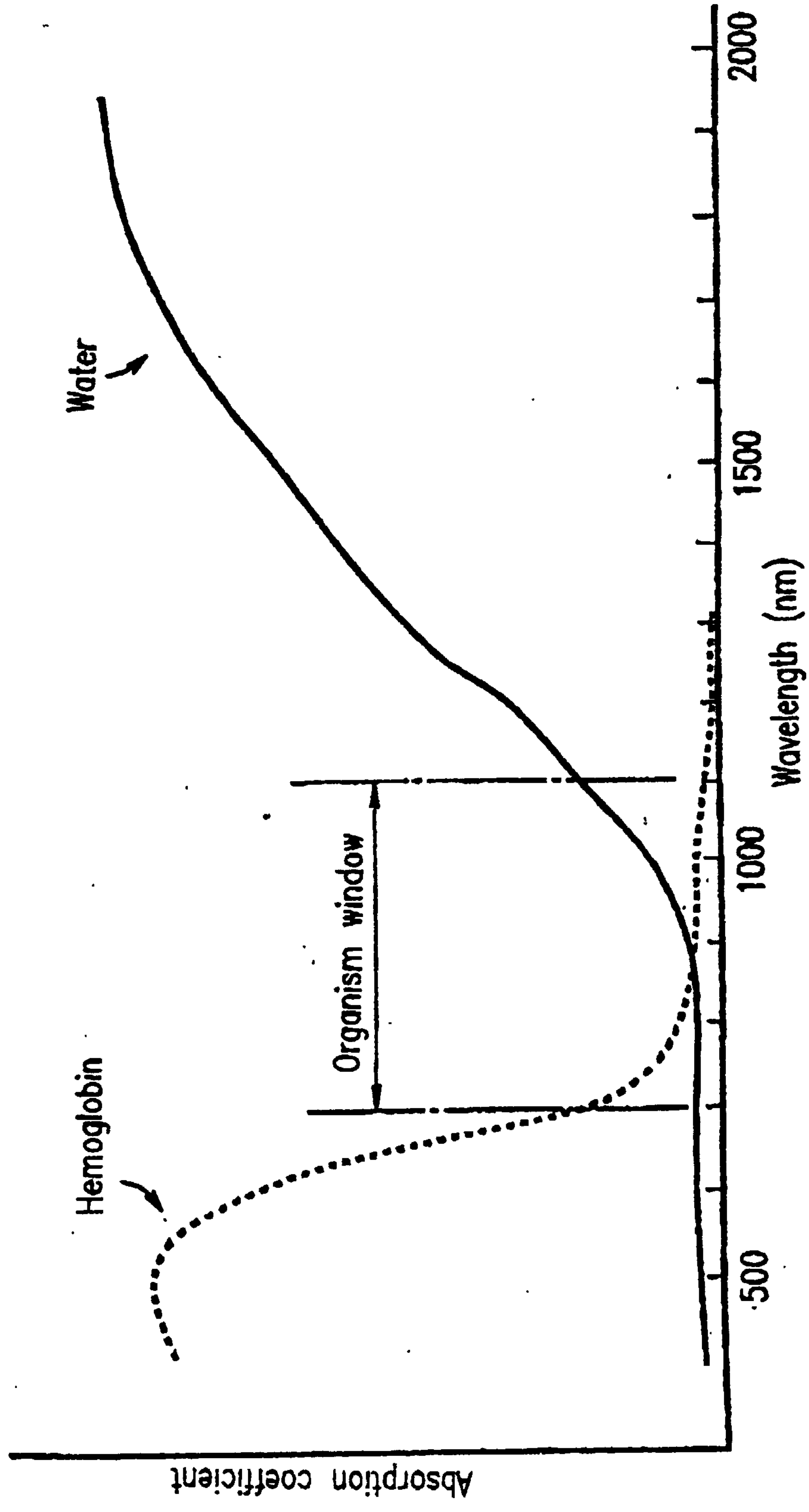
Water
Hemoglobin
Organism window
Absorption coefficient
Wavelength (nm)
500
1000
1500
2000

FIG.5

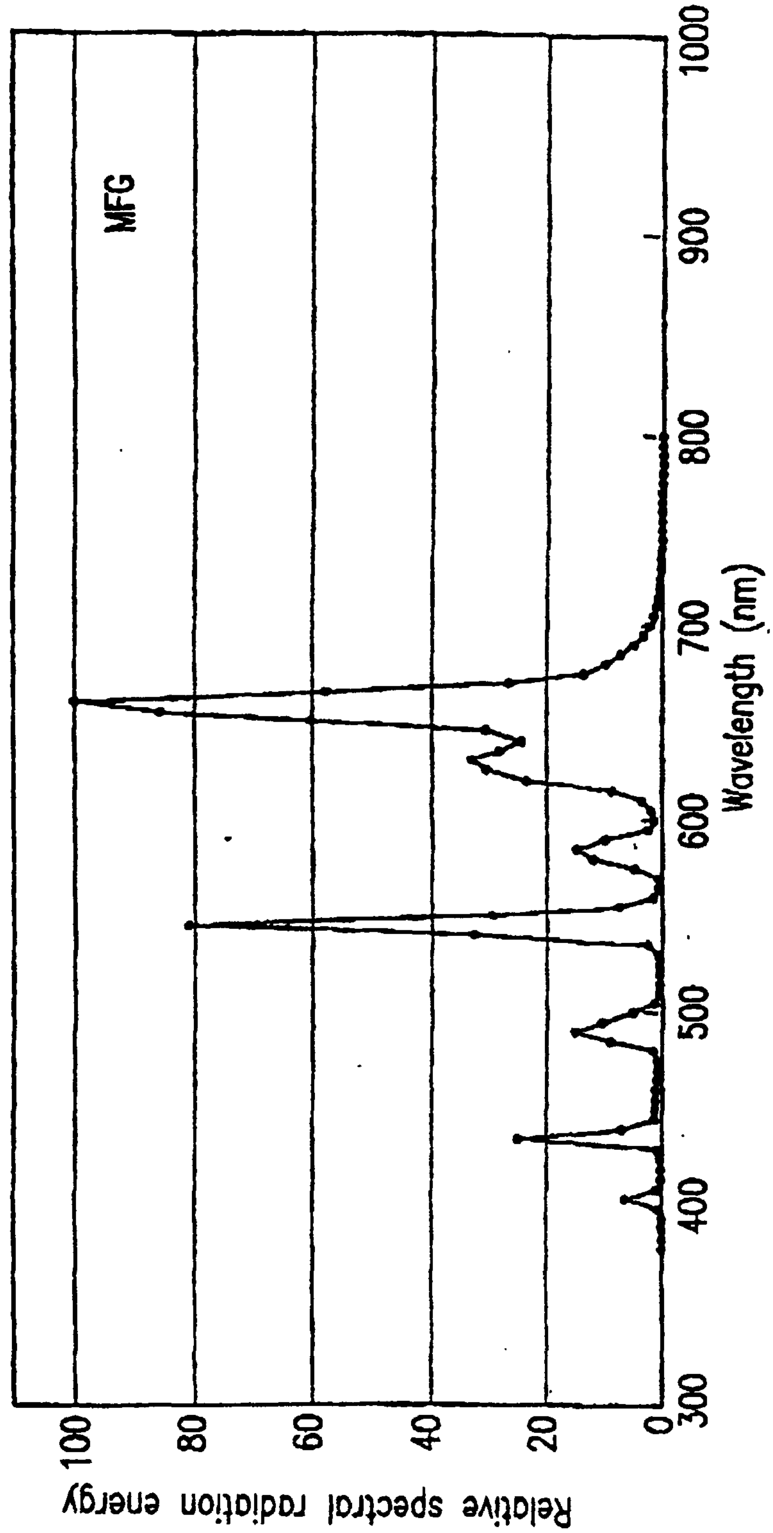
MFG
Relative spectral radiation energy
0
20
40
60
80
100
300
400
500
600
700
800
900
1000
Wavelength (nm)

*FIG.6*

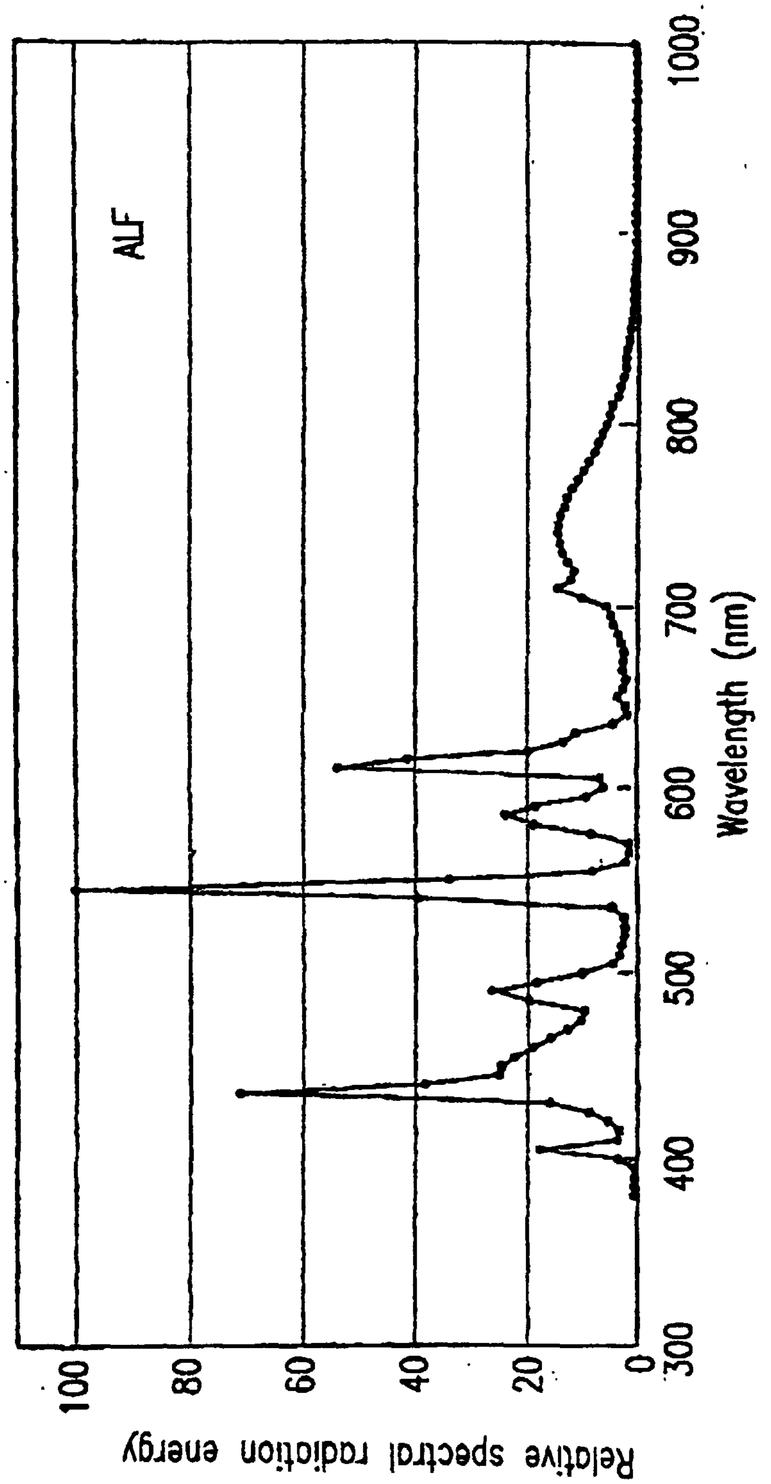
ALF
Relative spectral radiation energy
0
20
40
60
80
100
300
400
500
600
700
800
900
1000
Wavelength (nm)

FIG. 7

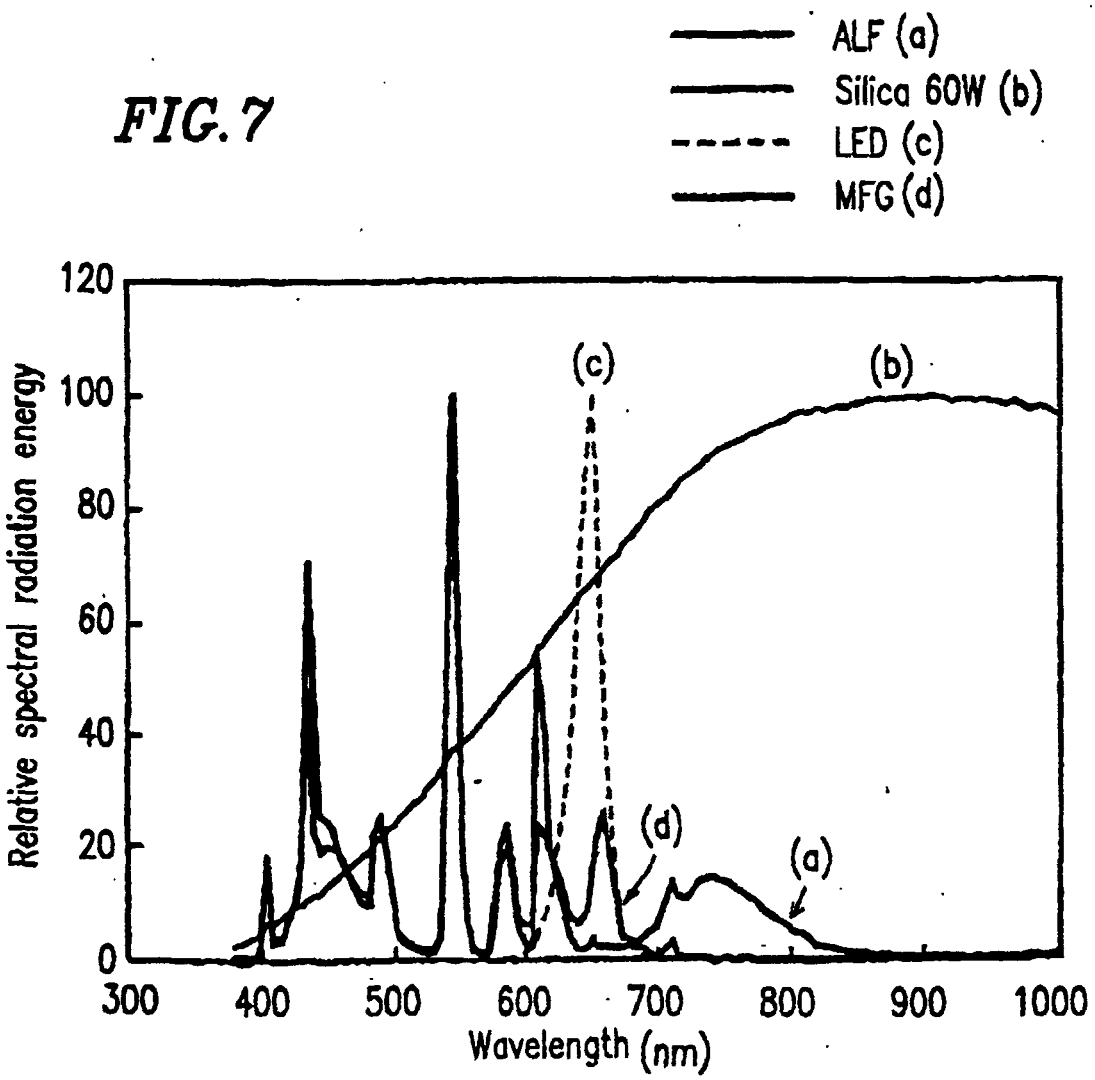
ALF (a)
Silica 60W (b)
LED (c)
MFG (d)
120
100
80
60
40
20
0
300
400
500
600
700
800
900
1000
Relative spectral radiation energy
Wavelength (nm)
(c)
(b)
(d)
(a)

FIG.8

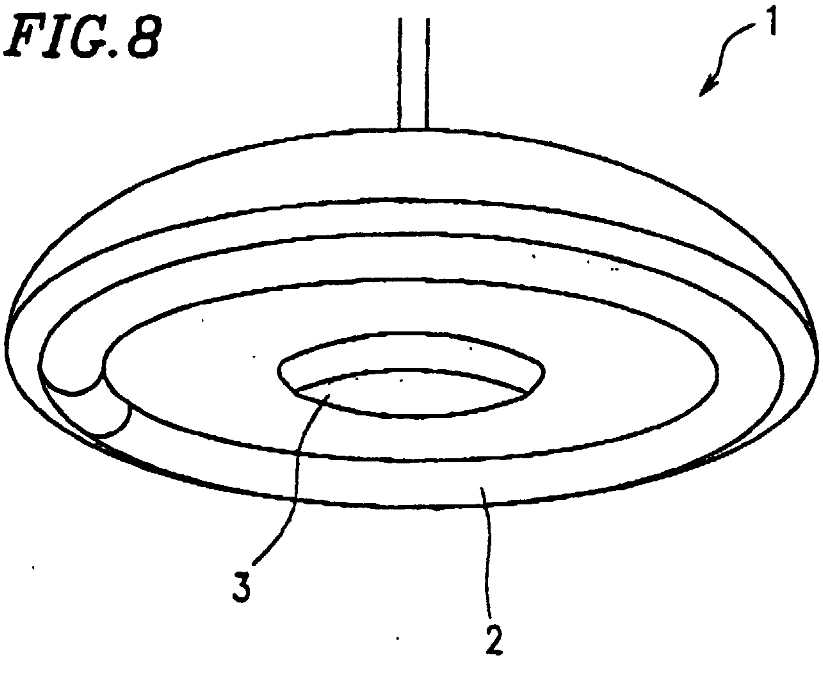

FIG. 9

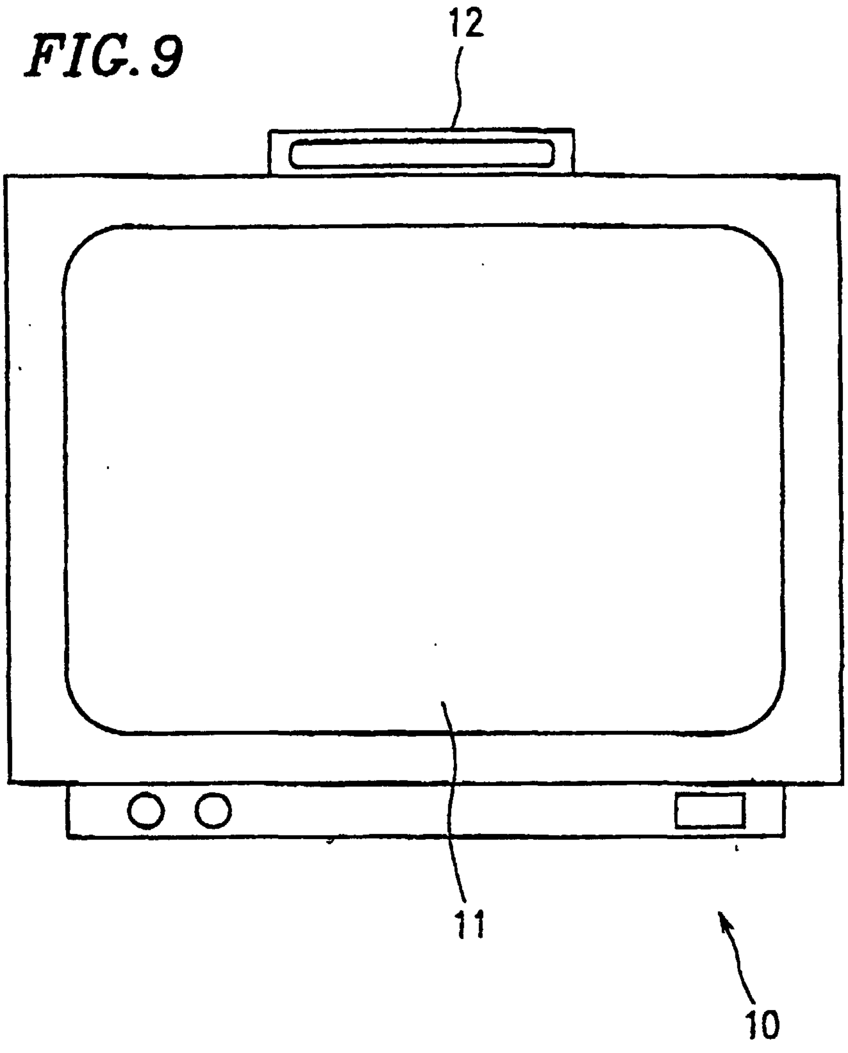

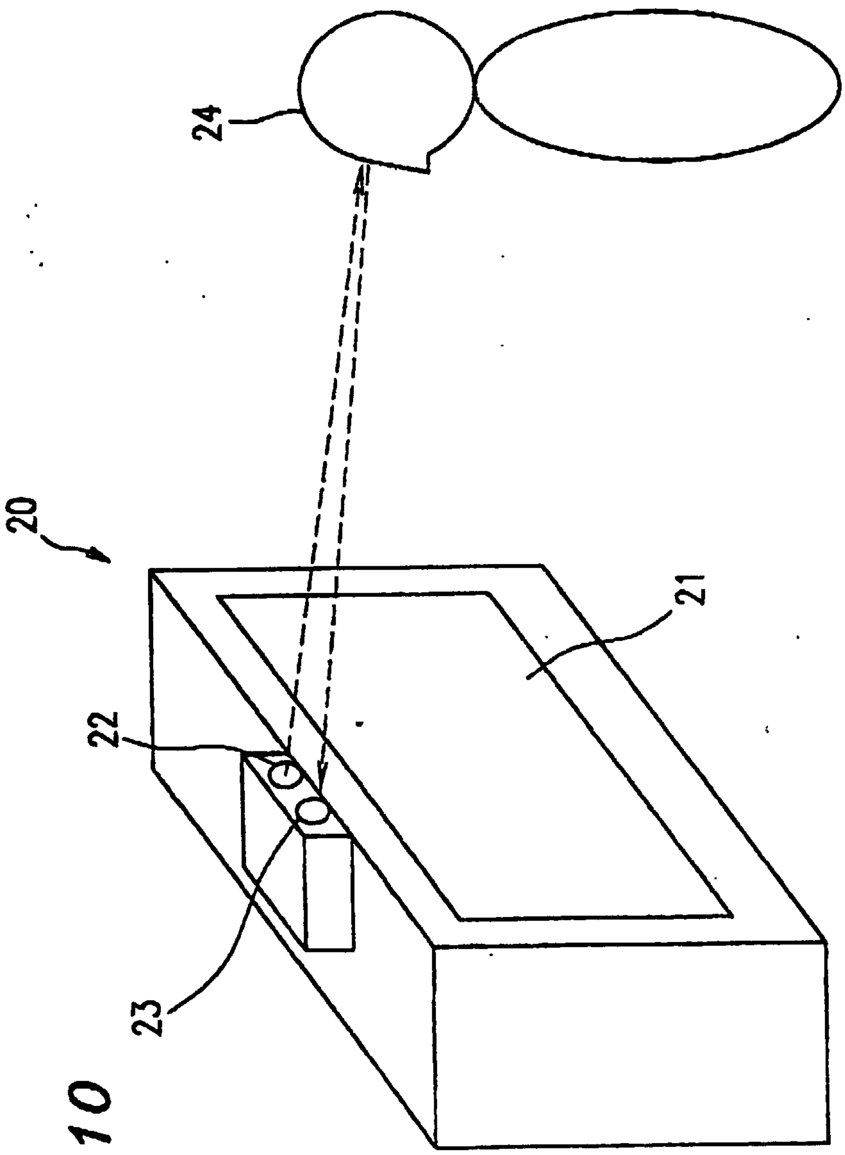


FIG. 10

# INTERNATIONAL SEARCH REPORT

International application No.
PCT/JP99/04507

A. CLASSIFICATION OF SUBJECT MATTER
Int.Cl[6] F21S1/00, F21V33/00, A61N5/06

According to International Patent Classification (IPC) or to both national classification and IPC

B. FIELDS SEARCHED

Minimum documentation searched (classification system followed by classification symbols)
Int.Cl[6] F21S1/00, F21V33/00, A61N5/06

Documentation searched other than minimum documentation to the extent that such documents are included in the fields searched

Jitsuyo Shinan Koho 1926-1996 Toroku Jitsuyo Shinan Koho 1994-1999
Kokai Jitsuyo Shinan Koho 1971-1999 Jitsuyo Shinan Toroku Koho 1996-1999

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP, 9-84888, A (Tsutomu Kamei), 31 March, 1997 (31. 03. 97) (Family: none) | 1-8, 12-16, 17-22, 24-28 |
| A | | 9-11, 23, 29-31 |
| Y | JP, 62-168322, A (Mitsubishi Electric Corp.), 24 July, 1987 (24. 07. 87) (Family: none) | 1-5, 7, 8, 10, 13-16 |
| Y | Microfilm of the specification and drawings annexed to the request of Japanese Utility Model Application No. 59-90915 (Laid-open No. 61-7803) (Nakakazu Sasaki), 17 January, 1986 (17. 01. 86) (Family: none) | 1-4, 6-8, 10, 13-16 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October, 1999 (26. 10. 99) | 2 November, 1999 (02. 11. 99) |
| Name and mailing address of the ISA/ Japanese Patent Office | Authorized officer |
| Facsimile No. | Telephone No. |

Form PCT/ISA/210 (second sheet) (July 1992)